# EUROPEAN PATENT APPLICATION

(11) **EP 4 674 853 A1**
(43) Date of publication of application: **07.01.2026**
(21) Application number: 24763791.1
(22) Date of filing: 22.02.2024
(51) Int. Cl.: C07D 495/04, H01L 27/146, H04N 25/70, H10K 30/30, H10K 30/60, H10K 39/32

(54) **COMPOUND, COMPOSITION, FILM, PHOTOELECTRIC CONVERSION ELEMENT AND CMOS IMAGE SENSOR**

(30) Priority: 02.03.2023 JP 2023031591; 09.06.2023 JP 2023095405; 27.12.2023 JP 2023221244
(71) Applicant: Mitsubishi Chemical Corporation, Tokyo 100-8251 (JP)
(72) Inventor: MIYATA Yasuo, Tokyo 100-8251 (JP)
(74) Representative: Hoffmann Eitle
(86) International application number: PCT/JP2024/006586
(87) International publication number: WO 2024/181311

(57) **Abstract**

Provided are a compound capable of lengthening an absorption wavelength while suppressing a dark current of a photoelectric conversion element, and a composition, a film, a photoelectric conversion element, and a CMOS image sensor using the compound. The compound of the present invention is represented by General Formula (1). In General Formula (1), X¹ to X⁸ are each independently a hydrogen atom, a cyano group, or the like, at least one of X¹ to X⁸ is a cyano group, Y¹ to Y⁴ are each independently a hydrogen atom, an alkyl group, or the like, at least one of Y¹ to Y⁴ is an alkyl group, an alkoxy group, or an ester group, Z¹ and Z² are each independently an oxygen atom or a dicyanomethylene group, and M is a carbon atom, a silicon atom, or a germanium atom, each of which is substituted with an alkyl group or an aryl group.

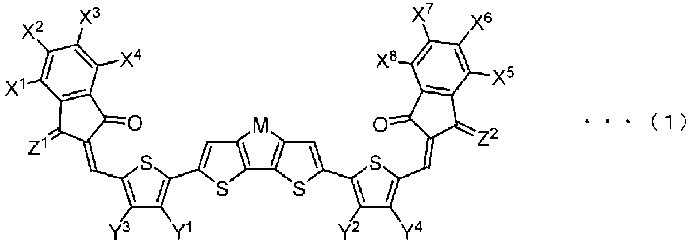

## Description

### Technical Field

The present invention relates to a compound which is suitable as semiconductor materials used in a photoelectric conversion element, a composition, a film, a photoelectric conversion element, and a CMOS image sensor.

Priority is claimed on Japanese Patent Application No. 2023-31591, filed on March 2, 2023, Japanese Patent Application No. 2023-95405, filed on June 9, 2023, and Japanese Patent Application No. 2023-221244, filed on December 27, 2023, the contents of which are incorporated herein by reference.

### Background Art

A CMOS image sensor including a photoelectric conversion element is used as an imaging element of a digital camera or smartphone, for example.

The CMOS image sensor includes an inorganic CMOS image sensor and an organic CMOS image sensor, and the inorganic CMOS image sensor using a silicon photodiode is generally used.

On the other hand, the organic CMOS image sensor can achieve both of realization of high resolution and a wide dynamic range and mounting of a global shutter that is less likely to cause image distortion by utilizing high light absorption capability of an organic thin film. As described above, it is said that the organic CMOS image sensor can solve the problem of achieving both a high dynamic range and mounting of a global shutter, which are difficult for the inorganic CMOS image sensor to achieve, and, therefore, a material suitable for the organic CMOS image sensor is required.

In addition, in a photoelectric conversion element included in the inorganic CMOS image sensor (hereinafter, also referred to as "inorganic photoelectric conversion element"), an inexpensive silicon semiconductor is generally used for an optical response when the absorption wavelength is up to 1000 nm, but an extremely expensive indium gallium arsenide (InGaAs) semiconductor is used when the absorption wavelength is 1000 nm or more. Therefore, a semiconductor material that is inexpensive and can be used in a long wavelength region is required, and an organic semiconductor material (hereinafter, also referred to as "organic semiconductor material") has attracted attention as a candidate therefor.

In a photoelectric conversion element included in the organic CMOS image sensor (hereinafter, also referred to as "organic photoelectric conversion element"), it is possible to control photoelectric conversion capability and an absorption wavelength range by molecular design of a p-type semiconductor material and an n-type semiconductor material used in an organic thin film (photoelectric conversion layer) constituting the photoelectric conversion element. In recent years, high photoelectric conversion capability has been reported in an element using a non-fullerene acceptor as the n-type semiconductor material. In a photoelectric conversion element using a non-fullerene acceptor, the n-type semiconductor material mainly plays a role of controlling the absorption wavelength range.

As the n-type semiconductor material (light absorbing material and electron transporting material), a compound having an electron acceptor (A) moiety and an electron donor (D) moiety, a so-called A-D-A type compound, is known. The absorption wavelength of the A-D-A type compound can be designed by reducing a HOMO-LUMO gap through selection of an electron-withdrawing property of the A moiety and an electron-donating property of the D moiety.

As the A-D-A type compound, an A-D¹-D-D²-A type compound is known, in which cyclopentadithiophene is used as a central donor (D) moiety, and the D moiety is sandwiched between thiophene rings (D¹) and (D²) substituted with specific substituents. For example, NPTL 1 discloses a compound represented by the following formula (a), which is an A-D'-D-D'-A type compound, a compound represented by the following formula (b), which is an A-D'-D-D"-A type compound, and a compound represented by the following formula (c), which is an A-D"-D-D"-A type compound. Here, D' represents a thiophene ring substituted with an alkoxy group, and D" represents a thiophene ring substituted with an alkyl group. According to NPTL 1, a compound represented by the following formula (a) can achieve the greatest lengthening of the absorption wavelength. The compound represented by the following formula (a) is a compound in which the alkyl group bonded to the thiophene ring in the D" moiety of the compound represented by the following formula (b) or (c) is substituted with an alkoxy group, which is a strong electron-donating group, to form a D' moiety.

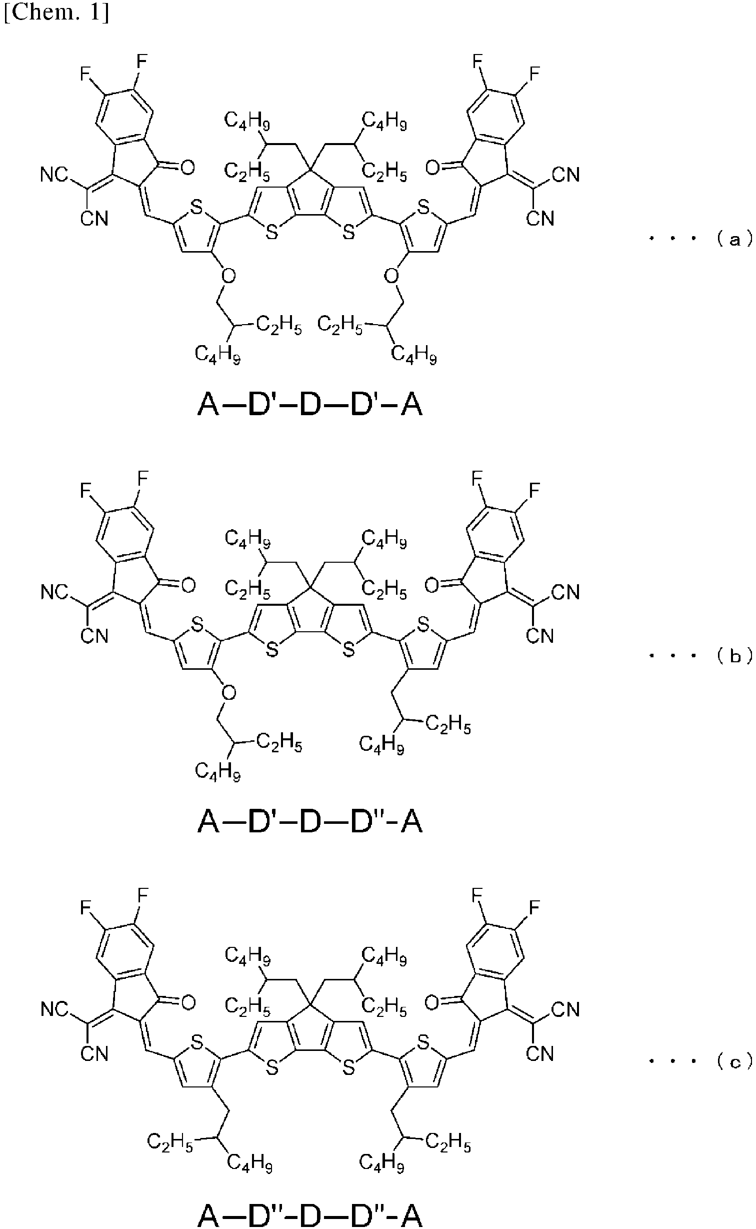

### Citation List

### Non-Patent Literature

NPTL 1: Jaewon Lee and other 12 persons, "ACS Energy Lett.", 2019, Vol. 4, p. 1401-1409

### Summary of Invention

### Technical Problem

In the organic semiconductor material, when the absorption wavelength is lengthened, a trade-off occurs in which a dark current increases and sensor sensitivity decreases. This is because the probability of carrier generation due to thermal excitation increases due to a decrease in HOMO-LUMO gap of the organic semiconductor material. Therefore, as the A-D-A type compound used in the photoelectric conversion element, a compound capable of realizing lengthening of the absorption wavelength of the organic semiconductor material while suppressing the dark current of the photoelectric conversion element is required.

An object of the present invention is to provide a compound capable of lengthening an absorption wavelength while suppressing a dark current of a photoelectric conversion element, and a composition, a film, a photoelectric conversion element, and a CMOS image sensor using the compound.

### Solution to Problem

In view of the above issues, the present inventor has conducted studies on a compound capable of lengthening an absorption wavelength while suppressing a dark current of a photoelectric conversion element. Specifically, the inventor has studied substituents in the A moiety of the A-D¹-D-D²-A type compound as shown in NPTL 1. As a result, the inventor has found that, by substituting a terminal of the A moiety with a cyano group, lengthening the absorption wavelength can be achieved while suppressing the dark current of the photoelectric conversion element, and has completed the present invention.

That is, the present invention includes the following aspects.
[1] A compound represented by General Formula (1): 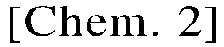 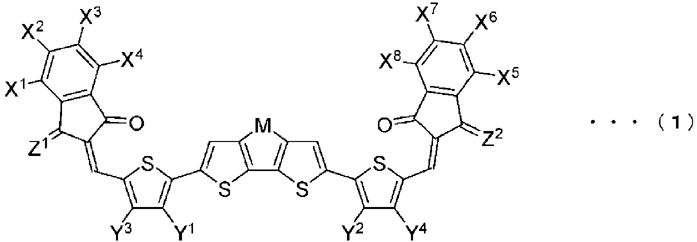 where
   X¹ to X⁸ are each independently a hydrogen atom, a chlorine atom, a fluorine atom, a bromine atom, or a cyano group, and at least one of X¹ to X⁸ is a cyano group,
   Y¹ to Y⁴ are each independently a hydrogen atom, an alkyl group, an alkoxy group, or an ester group, and at least one of Y¹ to Y⁴ is an alkyl group, an alkoxy group, or an ester group,
   Z¹ and Z² are each independently an oxygen atom or a dicyanomethylene group, and
   M is a carbon atom substituted with an alkyl group or an aryl group, a silicon atom substituted with an alkyl group or an aryl group, or a germanium atom substituted with an alkyl group or an aryl group.
[2] The compound according to [1], wherein, in General Formula (1), Z¹ and Z² are dicyanomethylene groups.
[3] The compound according to [1] or [2], wherein, in General Formula (1),
   X¹, X⁴, X⁵ and X⁸ are hydrogen atoms, and
   X², X³, X⁶ and X⁷ are cyano groups.
[4] The compound according to any one of [1] to [3], wherein, in General Formula (1), M is a carbon atom substituted with an alkyl group or an aryl group.
[5] The compound according to any one of [1] to [4], wherein, in General Formula (1),
   one of Y¹ and Y³ is a hydrogen atom and the other is an alkyl group, an alkoxy group, or an ester group, and
   one of Y² and Y⁴ is a hydrogen atom and the other is an alkyl group, an alkoxy group, or an ester group.
[6] The compound according to any one of [1] to [5], wherein, in General Formula (1),
   Y³ and Y⁴ are hydrogen atoms, and
   Y¹ and Y² are each independently an alkyl group, an alkoxy group, or an ester group.
[7] The compound according to any one of [1] to [6], wherein, in General Formula (1),
   X¹, X⁴, X⁵ and X⁸ are hydrogen atoms,
   X², X³, X⁶ and X⁷ are cyano groups,
   Y³ and Y⁴ are hydrogen atoms,
   Y¹ and Y² are alkoxy groups,
   Z¹ and Z² are dicyanomethylene groups, and
   M is a carbon atom substituted with an alkyl group.
[8] The compound according to any one of [1] to [5], wherein, in General Formula (1),
   Y² and Y³ are hydrogen atoms, and
   Y¹ and Y⁴ are each independently an alkyl group, an alkoxy group, or an ester group.
[9] The compound according to any one of [1] to [5] and [8], wherein, in General Formula (1),
   X¹, X⁴, X⁵ and X⁸ are hydrogen atoms,
   X², X³, X⁶ and X⁷ are cyano groups,
   Y² and Y³ are hydrogen atoms,
   Y¹ and Y⁴ are each independently an alkyl group or an alkoxy group,
   Z¹ and Z² are dicyanomethylene groups, and
   M is a carbon atom substituted with an alkyl group.
[10] A composition containing the compound described in any one of [1] to [9].
[11] A film containing the compound described in any one of [1] to [9].
[12] A photoelectric conversion element including the film described in [11].
[13] A CMOS image sensor including the photoelectric conversion element described in [12].

### Advantageous Effects of Invention

According to the present invention, it is possible to provide a compound capable of lengthening an absorption wavelength while suppressing a dark current of a photoelectric conversion element, and a composition, a film, a photoelectric conversion element, and a CMOS image sensor using the compound.

### Brief Description of Drawings

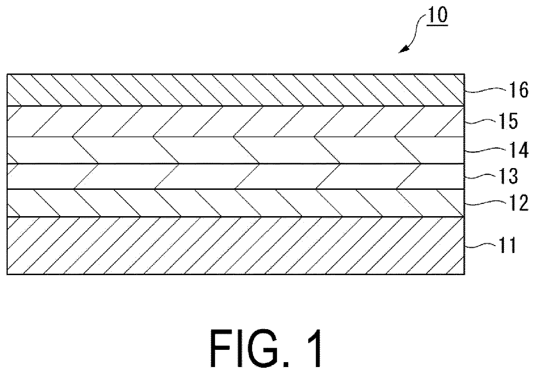

FIG. 1 is a cross-sectional view schematically showing an example of an embodiment of a photoelectric conversion element of the present invention.

### Description of Embodiments

Hereinafter, the present invention will be described in more detail with reference to preferred embodiments of the present invention, but the following description is an example of an embodiment of the present invention, and the present invention is not limited to the following description unless it goes beyond the gist of the present invention.

In the present specification, "from ... to ..." indicating a numerical range means that numerical values described before and after the term "to" are included as a lower limit value and an upper limit value.

### [Compound]

The compound of the present invention is a compound represented by General Formula (1) (hereinafter, also referred to as "Compound (1)"; the same applies hereinafter).

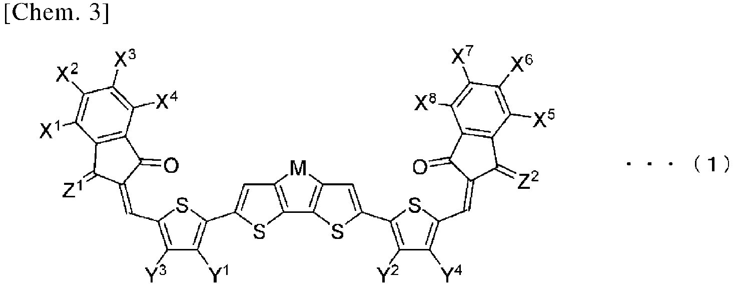

In General Formula (1), X¹ to X⁸ are each independently a hydrogen atom, a chlorine atom, a fluorine atom, a bromine atom, or a cyano group, and at least one of X¹ to X⁸ is a cyano group; Y¹ to Y⁴ are each independently a hydrogen atom, an alkyl group, an alkoxy group, or an ester group, and at least one of Y¹ to Y⁴ is an alkyl group, an alkoxy group, or an ester group; Z¹ and Z² are each independently an oxygen atom or a dicyanomethylene group; and M is a carbon atom substituted with an alkyl group or an aryl group, a silicon atom substituted with an alkyl group or an aryl group, or a germanium atom substituted with an alkyl group or an aryl group.

The compound of the present invention, which is an A-D¹-D-D²-A type compound with a terminal of an A moiety substituted with a cyano group, can lengthen an absorption wavelength while suppressing a dark current of a photoelectric conversion element.

The reason why the compound of the present invention can lengthen the absorption wavelength while suppressing the dark current of the photoelectric conversion element is not clear, but is presumed as follows.

The compound of the present invention has substituents on one atom at the center of a D moiety in directions projecting above and below a *π-*conjugated plane, and, on the other hand, has substituents extending in the same direction on the *π*-conjugated plane in a D¹ moiety and a D² moiety. Therefore, the compound of the present invention tends to have a *π*-stack structure in which substituents projecting above and below the *π*-conjugated plane do not overlap each other but slip between molecules. On the other hand, since the substituents on the intermolecular *π*-conjugated plane have a lamellar structure, the compound of the present invention is probably a compound capable of having a densely packed structure.

Moreover, in the compound of the present invention, the terminal of the A moiety of the A-D¹-D-D²-A type compound having a densely packed structure is substituted with a cyano group which is more likely to develop an intermolecular interaction than the known A-D¹-D-D²-A type compound. Therefore, it is probable that a densely packed structure of the A-D¹-D-D²-A type compounds is promoted also in a direction orthogonal to the lamellar structure (that is, a long axis direction of a *π*-conjugated skeleton of the molecule), and that charge separation between the n-type semiconductor material and the p-type semiconductor material under a dark condition can be suppressed.

Probably by this mechanism. the compound of the present invention can achieve also suppression of an increase in dark current due to a densely packed structure in addition to the lengthening of the absorption wavelength due to a strong electron-withdrawing property by substituting the terminal of the A moiety with a cyano group in addition to the packed structure due to the characteristic direction of the substituents of a D¹-D-D² moiety in the A-D¹-D-D²-A type compound.

US 11233207 lists cyano groups together with fluorine atoms, chlorine atoms and the like as substituents that can be substituted at the terminal of the A moiety, but does not disclose cyano group-substituted compounds as specific examples since synthesis of the cyano group-substituted compounds is more complicated than that of halogen-substituted compounds.

In General Formula (1), X¹ to X⁸ are each independently a hydrogen atom, a chlorine atom, a fluorine atom, a bromine atom or a cyano group, and at least one of X¹ to X⁸ is a cyano group. It is likely that Compound (1) can maintain and improve the electron-withdrawing property of the A moiety when X¹ to X⁸ are hydrogen atoms, chlorine atoms, fluorine atoms, bromine atoms, or cyano groups. Furthermore, when at least one of X¹ to X⁸ is a cyano group, the absorption wavelength can be lengthened due to the strong electron-withdrawing property, and, besides, an increase in dark current due to a densely packed structure can be suppressed. In particular, X², X³, X⁶ and X⁷ are preferably cyano groups from the viewpoint of further promoting a densely packed structure of the compounds. X¹, X⁴, X⁵ and X⁸ are preferably hydrogen atoms from the viewpoint of ease of synthesis. That is, it is preferable that X¹, X⁴, X⁵ and X⁸ of Compound (1) be hydrogen atoms and that X², X³, X⁶ and X⁷ be cyano groups.

In another embodiment, X¹, X⁴, X⁵ and X⁸ of Compound (1) may be hydrogen atoms, X², X³, X⁶ and X⁷ may each independently be a hydrogen atom, a chlorine atom, a fluorine atom, a bromine atom or a cyano group, and one to three, preferably two or three, of X², X³, X⁶ and X⁷ may be cyano groups. In this case, the rest of X², X³, X⁶ and X⁷ are preferably hydrogen atoms, and one or two of X², X³, X⁶ and X⁷ are more preferably hydrogen atoms.

In General Formula (1), Y¹ to Y⁴ are each independently a hydrogen atom, an alkyl group, an alkoxy group, or an ester group, at least one of Y¹ to Y⁴ is an alkyl group, an alkoxy group, or an ester group.

The number of carbon atoms in the alkyl groups of Y¹ to Y⁴ is preferably small from the viewpoint of electroconductivity of the material. Therefore, the number of carbon atoms in the alkyl groups of Y¹ to Y⁴ is preferably 30 or less, more preferably 20 or less, still more preferably 15 or less, and particularly preferably 10 or less. Also, the number of carbon atoms in the alkyl groups of Y¹ to Y⁴ is preferably 2 or more, more preferably 4 or more, still more preferably 6 or more, and particularly preferably 8 or more. The above upper and lower limits can be combined in any manner. For example, the number of carbon atoms may be from 2 to 30, from 4 to 20, from 6 to 15, or from 8 to 10.

The alkyl groups of Y¹ to Y⁴ may be chain or cyclic. In a case where the alkyl group is chain, the alkyl group may be linear or branched. From the viewpoint of the ease of synthesis, a linear or branched alkyl group in which a carbon atom bonded to a thiophene ring is a primary carbon atom is preferable. From the viewpoint of solubility of the material, a branched alkyl group in which a carbon atom bonded to a thiophene ring is a primary carbon atom, or a linear, branched, or cyclic alkyl group in which a carbon atom bonded to a thiophene ring is a secondary carbon atom is preferable. From the viewpoint of the ease of synthesis and the solubility, a branched alkyl group in which the carbon atom bonded to the thiophene ring is a primary carbon atom is still more preferable.

The number of carbon atoms in the alkoxy groups of Y¹ to Y⁴ is preferably small from the viewpoint of the electroconductivity of the material. Therefore, the number of carbon atoms in the alkoxy groups of Y¹ to Y⁴ is preferably 30 or less, more preferably 20 or less, still more preferably 15 or less, and particularly preferably 10 or less. Also, the number of carbon atoms in the alkoxy groups of Y¹ to Y⁴ is preferably 2 or more, more preferably 4 or more, still more preferably 6 or more, and particularly preferably 8 or more. The above upper and lower limits can be combined in any manner. For example, the number of carbon atoms may be from 2 to 30, from 4 to 20, from 6 to 15, or from 8 to 10.

The alkoxy group has a structure in which an alkyl group is bonded to an oxygen atom, and the alkyl group bonded to the oxygen atom may be chain or cyclic. In a case where the alkyl group bonded to the oxygen atom is chain, the alkyl group may be linear or branched. From the viewpoint of the ease of synthesis, a linear or branched alkyl group in which the carbon atom bonded to the oxygen atom is a primary carbon atom is preferable. From the viewpoint of the solubility of the material, a branched alkyl group in which the carbon atom bonded to the oxygen atom is a primary carbon atom, or a linear, branched, or cyclic alkyl group in which the carbon atom bonded to the oxygen atom is a secondary carbon atom is preferable, a linear, branched, or cyclic alkyl group in which the carbon atom bonded to the oxygen atom is a secondary carbon atom is more preferable, and a linear or branched alkyl group in which the carbon atom bonded to the oxygen atom is a secondary carbon atom is still more preferable.

The ester groups of Y¹ to Y⁴ are, for example, monovalent groups having an ester bond. Specifically, the monovalent groups are, for example, groups represented by General Formula (i).

-COO-R¹ ... (i)

In General Formula (i), R¹ is an alkyl group or an aryl group.

The number of carbon atoms in the alkyl group of R¹ is preferably small from the viewpoint of the electroconductivity of the material. Therefore, the number of carbon atoms in the alkyl group of R¹ is preferably 30 or less, more preferably 20 or less, still more preferably 15 or less, and particularly preferably 10 or less. Also, the number of carbon atoms in the alkyl group of R¹ is preferably 1 or more, more preferably 4 or more, still more preferably 6 or more, and particularly preferably 8 or more. The above upper and lower limits can be combined in any manner. For example, the number of carbon atoms may be from 2 to 30, from 4 to 20, from 6 to 15, or from 8 to 10.

The alkyl group of R¹ may be chain or cyclic. In a case where the alkyl group is chain, the alkyl group may be linear or branched. From the viewpoint of the ease of synthesis, a linear or branched alkyl group in which the carbon atom bonded to the oxygen atom is a primary carbon atom is preferable. From the viewpoint of the solubility of the material, a branched alkyl group in which the carbon atom bonded to the oxygen atom is a primary carbon atom, or a linear, branched, or cyclic alkyl group in which the carbon atom bonded to the oxygen atom is a secondary carbon atom is preferable, a linear, branched, or cyclic alkyl group in which the carbon atom bonded to the oxygen atom is a secondary carbon atom is more preferable, and a linear or branched alkyl group in which the carbon atom bonded to the oxygen atom is a secondary carbon atom is still more preferable.

The number of carbon atoms in the aryl group of R¹ is preferably small from the viewpoint of the electroconductivity of the material. Therefore, the number of carbon atoms in the aryl group of R¹ is preferably 18 or less, more preferably 12 or less, still more preferably 10 or less, and particularly preferably 6. A lower limit of the number of carbon atoms in the aryl group of R¹ is 6. For example, the number of carbon atoms may be from 6 to 18, from 6 to 12, from 6 to 10, or 6.

The aryl group of R¹ may or may not have a substituent. That is, the aryl group of R¹ is an unsubstituted or substituted aryl group. Examples of the substituent include an alkyl group, an alkoxy group, a hydroxy group, and an amino group.

Y¹ to Y⁴ may be the same or different. In particular, from the viewpoint of easily taking a lamellar structure in a film produced by using the compound of the present invention, it is preferable that one of Y¹ and Y³ be a hydrogen atom and that the other be an alkyl group, an alkoxy group, or an ester group, and that one of Y² and Y⁴ be a hydrogen atom and that the other be an alkyl group, an alkoxy group, or an ester group.

From the viewpoint of the ease of synthesis, it is preferable that Y¹ to Y⁴ be symmetrical with respect to M, that is, Y¹ and Y² be the same, and that Y³ and Y⁴ be the same. Especially, from the viewpoint of lengthening the absorption wavelength, it is more preferable that Y³ and Y⁴ be hydrogen atoms and that Y¹ and Y² be alkyl groups, alkoxy groups, or ester groups, and it is still more preferable that Y³ and Y⁴ be hydrogen atoms and that Y¹ and Y² be alkoxy groups.

In addition, as another embodiment, from the viewpoints that orientations of the substituents become parallel and that a lamellar structure is more easily taken, it is preferable that Y¹ to Y⁴ be asymmetric with respect to M, i.e., Y² and Y³ are hydrogen atoms, that Y¹ and Y⁴ be each independently an alkyl group, an alkoxy group or an ester group, it is more preferable that Y² and Y³ be hydrogen atoms, and that Y¹ and Y⁴ be each independently an alkyl group or an alkoxy group, and it is still more preferable that Y² and Y³ be hydrogen atoms, that one of Y¹ and Y⁴ be an alkyl group, and that the other be an alkoxy group.

In General Formula (1), Z¹ and Z² are each independently an oxygen atom or a dicyanomethylene group.

It is likely that Compound (1) can be an electron-withdrawing group of an acceptor moiety when Z¹ and Z² are oxygen atoms or dicyanomethylene groups. Z¹ and Z² are preferably both dicyanomethylene groups from the viewpoint of further enhancing the electron-withdrawing property.

In General Formula (1), M is a carbon atom substituted with an alkyl group or an aryl group, a silicon atom substituted with an alkyl group or an aryl group, or a germanium atom substituted with an alkyl group or an aryl group. It is likely that Compound (1) can have enhanced solubility when M is a carbon atom substituted with an alkyl group or an aryl group, a silicon atom substituted with an alkyl group or an aryl group, or a germanium atom substituted with an alkyl group or an aryl group.

The carbon atom substituted with an alkyl group or an aryl group is represented by General Formula (ii). The silicon atom substituted with an alkyl group or an aryl group is represented by General Formula (iii). The germanium atom substituted with an alkyl group or an aryl group is represented by General Formula (iv).

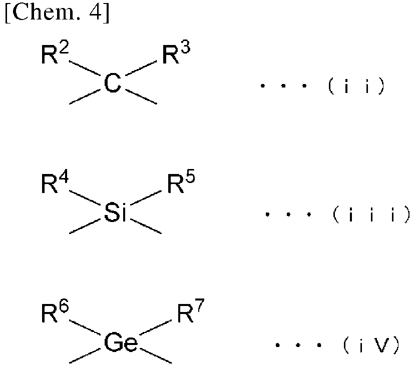

In General Formulae (ii) to (iv), R² to R⁷ are each independently an alkyl group or an aryl group.

Examples of the alkyl groups of R² to R⁷ include the alkyl group of R¹ exemplified above in the description of Y¹ to Y⁴.

Examples of the aryl groups of R² to R⁷ include the aryl group of R¹ exemplified above in the description of Y¹ to Y⁴.

From the viewpoint of the ease of synthesis of Compound (1), the two alkyl groups or aryl groups of the carbon atom, silicon atom and germanium atom are preferably the same.

From the viewpoint of lengthening the absorption wavelength, M is preferably a carbon atom substituted with an alkyl group or an aryl group, and more preferably a carbon atom substituted with an alkyl group.

The compound (1) is preferably a compound in which, in General Formula (1), X¹, X⁴, X⁵ and X⁸ are hydrogen atoms, X², X³, X⁶ and X⁷ are cyano groups, Y³ and Y⁴ are hydrogen atoms, Y¹ and Y² are alkoxy groups, Z¹ and Z² are dicyanomethylene groups, and M is a carbon atom substituted with an alkyl group.

In another aspect, Compound (1) is preferably a compound in which, in General Formula (1), X¹, X⁴, X⁵ and X³ are hydrogen atoms, X², X³, X⁶ and X⁷ are cyano groups, Y² and Y³ are hydrogen atoms, Y¹ and Y⁴ are each independently an alkyl group or an alkoxy group, Z¹ and Z² are dicyanomethylene groups, and M is a carbon atom substituted with an alkyl group, and, especially, a compound in which one of Y¹ and Y⁴ is an alkyl group and the other is an alkoxy group is more preferable, and a compound in which Y¹ is an alkoxy group, and Y⁴ is an alkyl group is most preferable.

Specific examples of Compound (1) include compounds represented by Formulae (1-1) to (1-60), but Compound (1) is not limited thereto. In the present specification, for example, a compound represented by the following formula (1-1) is also referred to as "Compound (1-1)". The same applies to compounds represented by the Formulae (1-2) to (1-60).

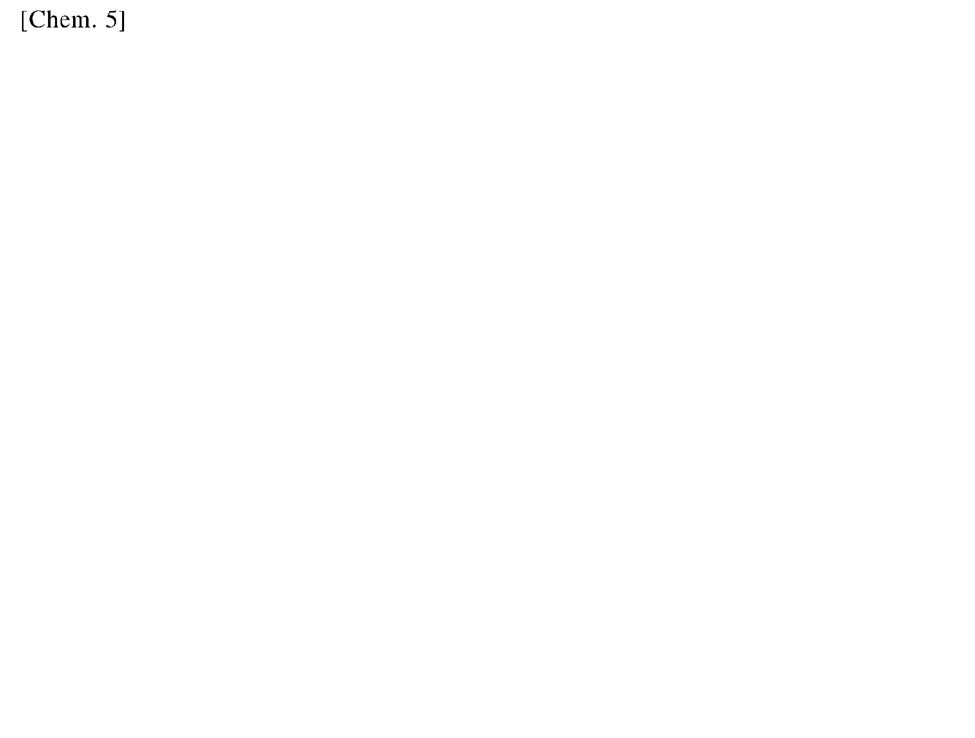

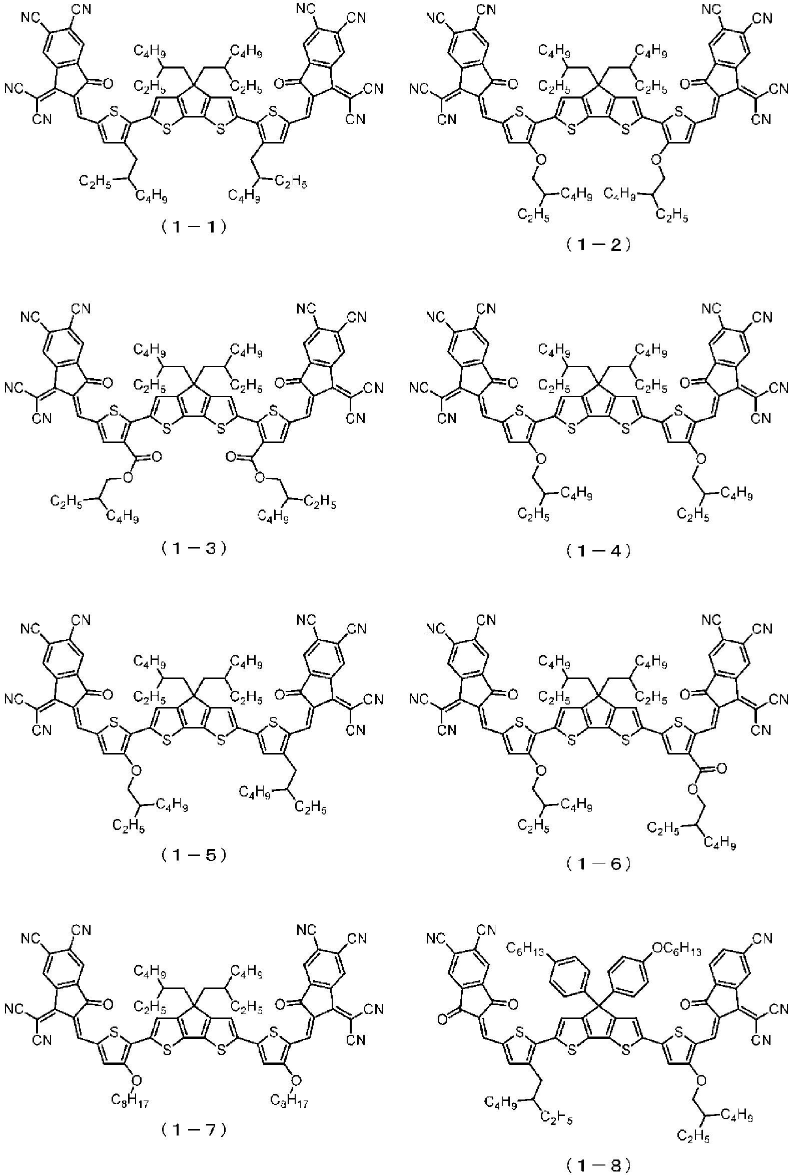

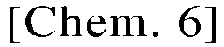

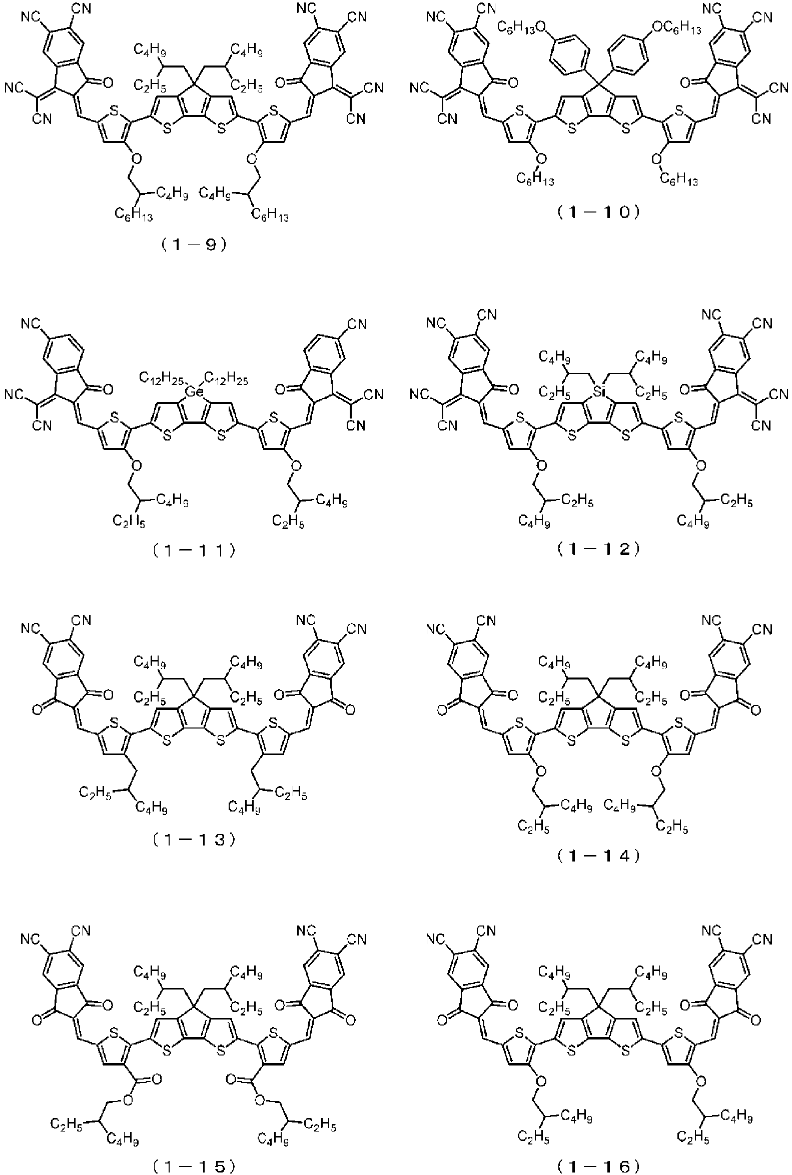

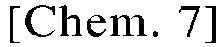

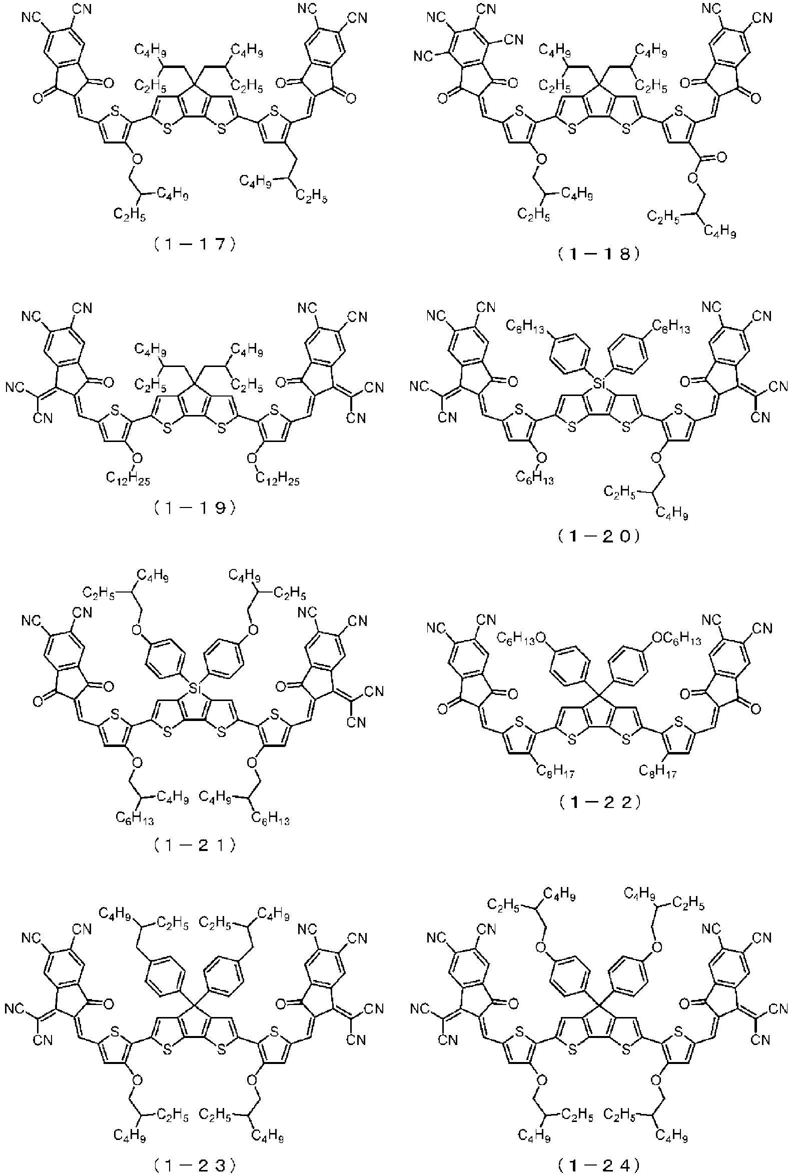

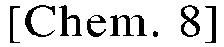

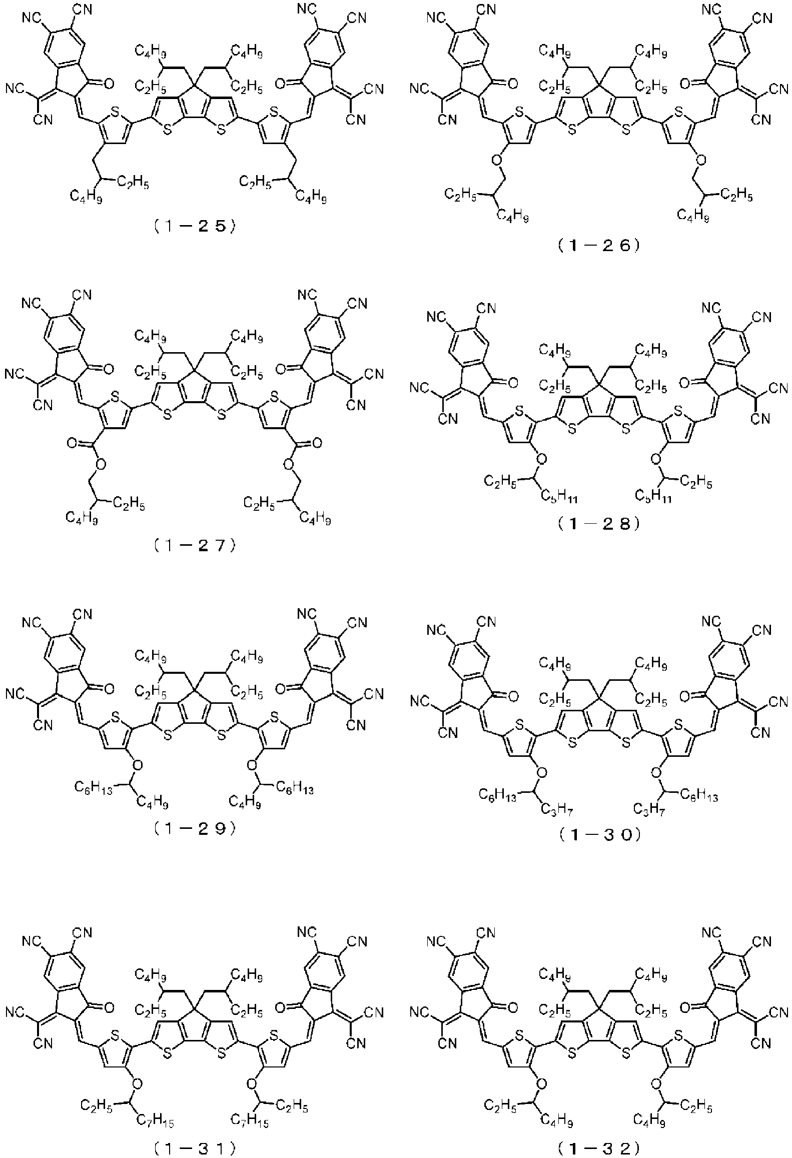

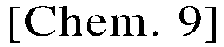

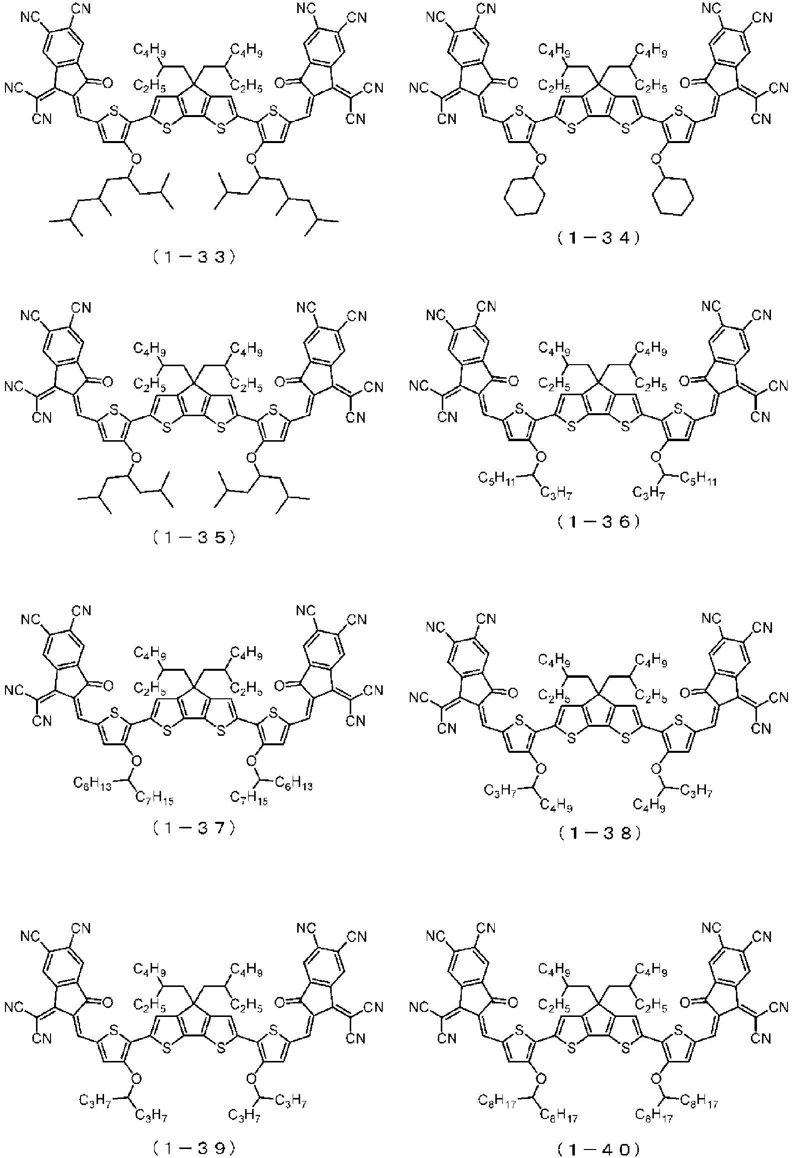

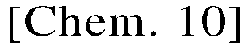

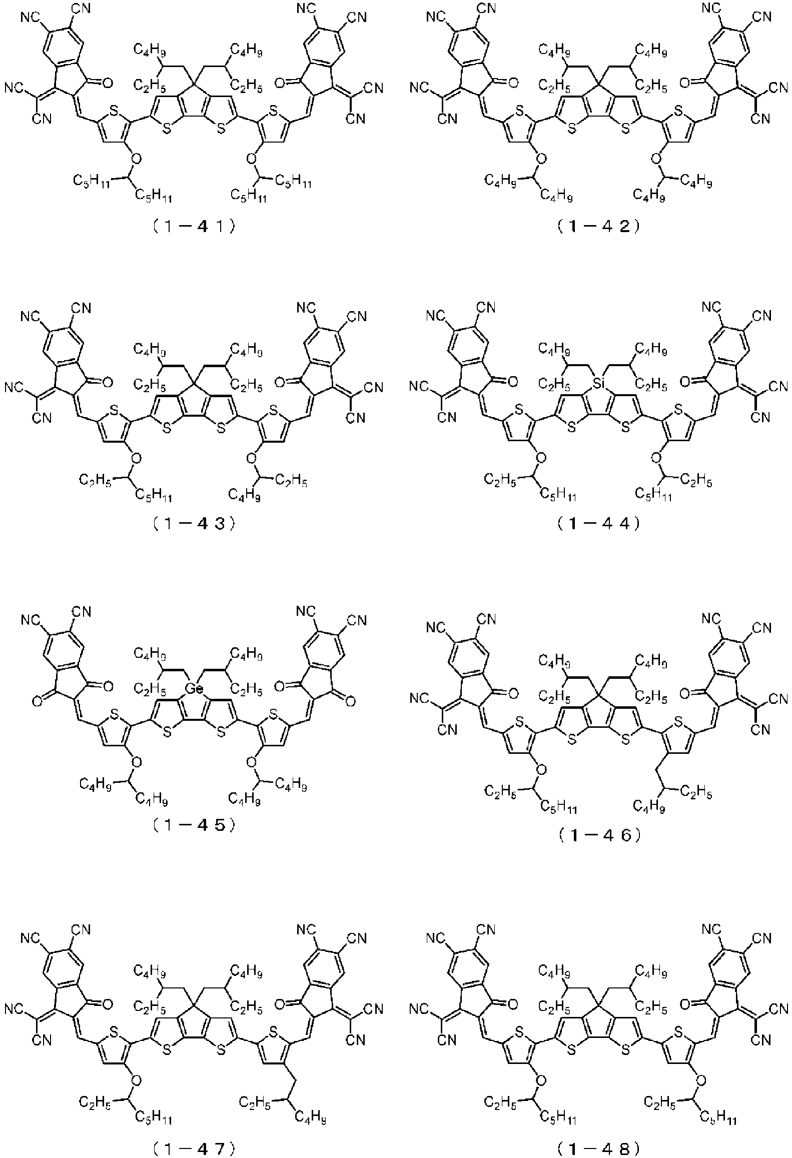

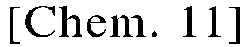

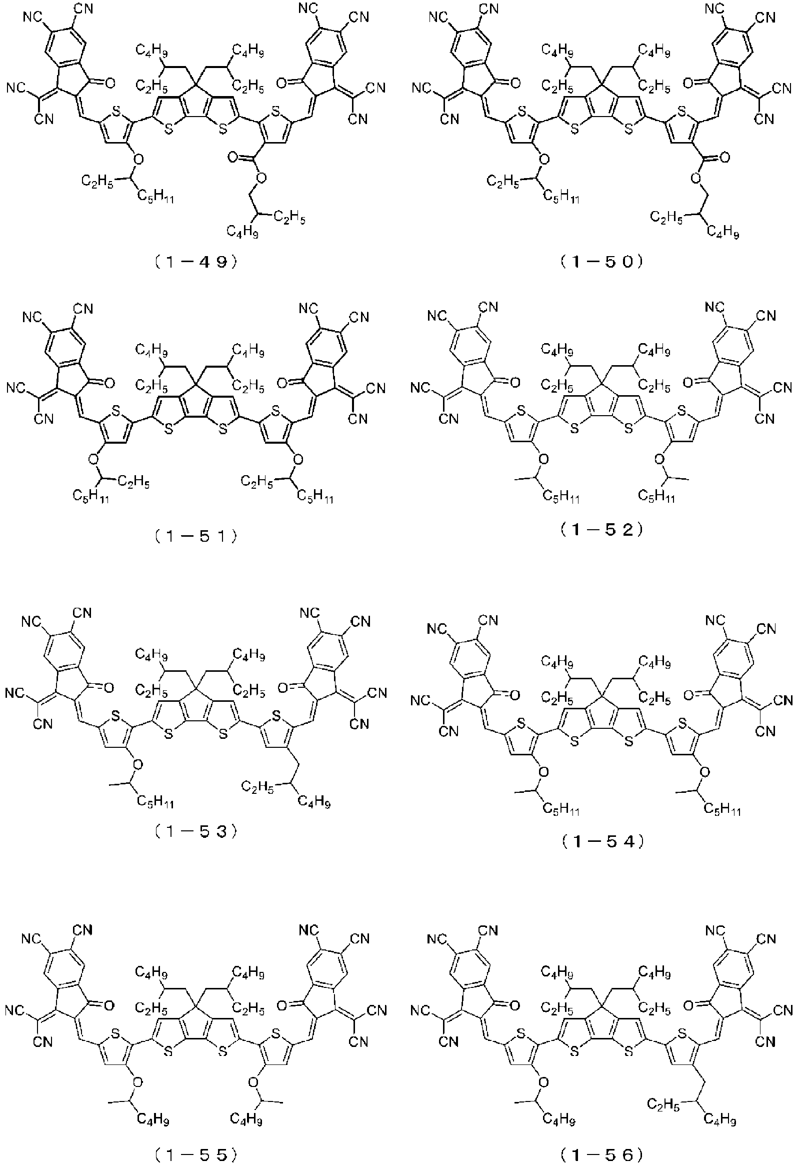

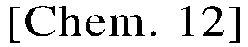

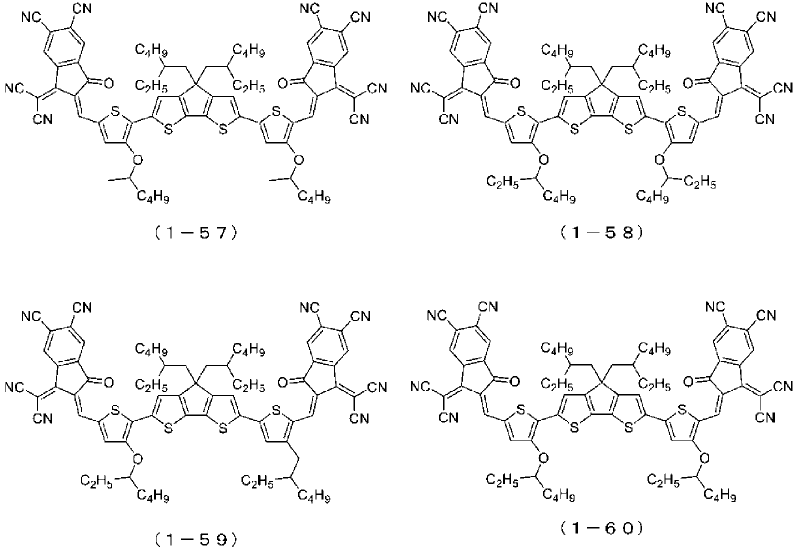

Among the compounds described above, a compound in which at least one of Y¹ to Y⁴ is an alkoxy group in which an alkyl group bonded to an oxygen atom is linear, branched, or cyclic, and a carbon atom bonded to an oxygen atom is a secondary carbon atom (that is, a compound in which an alkoxy group bonded to a thiophene ring is branched at 1-position) has particularly excellent solubility. Examples of such a compound include compounds (1-28) to (1-60).

The method for producing Compound (1) is not particularly limited, but as an example of the method for producing Compound (1), a method for producing Compound (A) will be specifically described. Y in Compound (A) corresponds to any of Y¹ to Y⁴ in Compound (1), and Z in Compound (A) corresponds to Z¹ or Z² in Compound (1).

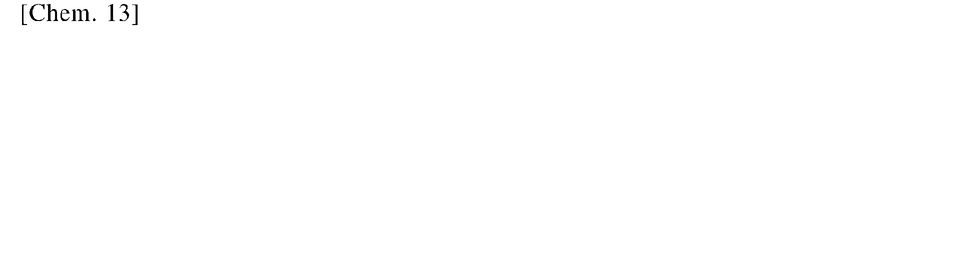

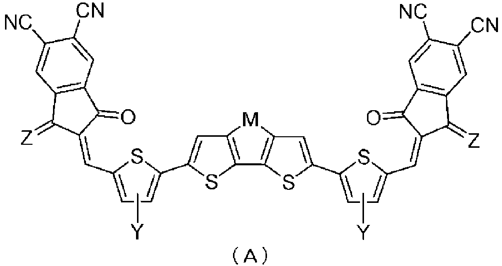

First, Compound (B) is reacted with lithium diisopropylamide (LDA) in a reaction solvent, and the reaction product is then further reacted with N,N-dimethylformamide to produce Compound (C).

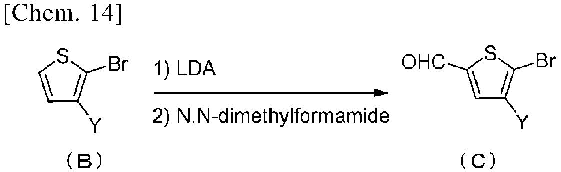

Here, Compound (B) may be synthesized by a known method, or a commercially available product may be used.

Charging ratios of the raw materials are preferably from 0.9 to 1.5 equivalents for LDA and preferably 0.9 equivalents or more for N,N-dimethylformamide, with respect to Compound (B). N-formylpiperidine may be used instead of N,N-dimethylformamide.

The reaction solvent is not particularly limited as long as it is a solvent that does not react with the raw material compound, and examples thereof include saturated aliphatic hydrocarbon solvents such as hexane; ether-based solvents such as tetrahydrofuran (THF), diethyl ether, cyclopentyl methyl ether, and methyl t-butyl ether; and aromatic hydrocarbon solvents such as toluene and xylene.

A reaction temperature is preferably from -78 to 50°C.

A reaction time is preferably from 10 minutes to 12 hours after addition of LDA and from 10 minutes to 12 hours after addition of N,N-dimethylformamide.

In the production of Compound (A), Compound (E) may be used in place of Compound (C). Compound (E) is produced, for example, as follows.

That is, first, 3-substituted thiophene is reacted with lithium diisopropylamide (LDA) in a reaction solvent, and then the reaction product is further reacted with 1,2-dibromo-1,1,2,2-tetrachloroethane to produce Compound (D).

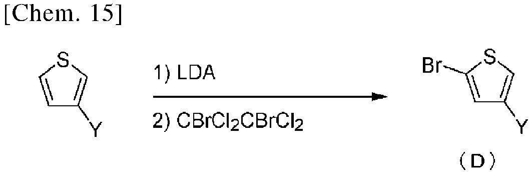

Here, the 3-substituted thiophene may be synthesized by a known method, or a commercially available product may be used.

The charging ratios of the raw materials are preferably from 0.9 to 1.5 equivalents for LDA and preferably 0.9 equivalents or more for 1,2-dibromo-1,1,2,2-tetrachloroethane, with respect to the 3-substituted thiophene.

The reaction solvent is not particularly limited as long as it is a solvent that does not react with the raw material compound, and examples thereof include saturated aliphatic hydrocarbon solvents such as hexane; ether-based solvents such as tetrahydrofuran (THF), diethyl ether, cyclopentyl methyl ether, and methyl t-butyl ether; and aromatic hydrocarbon solvents such as toluene and xylene.

A reaction temperature is preferably from -78 to 50°C.

A reaction time is preferably from 10 minutes to 12 hours after addition of LDA and from 10 minutes to 12 hours after addition of 1,2-dibromo-1,1,2,2-tetrachloroethane.

Next, Compound (D) is reacted with lithium diisopropylamide (LDA) in a reaction solvent, and the reaction product is then further reacted with N,N-dimethylformamide to produce Compound (E).

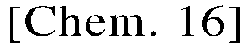

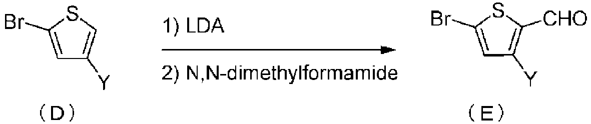

The charging ratios of the raw materials are preferably from 0.9 to 1.5 equivalents for LDA and preferably 0.9 equivalents or more for N,N-dimethylformamide with respect to Compound (D). N-formylpiperidine may be used instead of N,N-dimethylformamide.

The reaction solvent is not particularly limited as long as it is a solvent that does not react with the raw material compound, and examples thereof include saturated aliphatic hydrocarbon solvents such as hexane; ether-based solvents such as tetrahydrofuran (THF), diethyl ether, cyclopentyl methyl ether, and methyl t-butyl ether; and aromatic hydrocarbon solvents such as toluene and xylene.

A reaction temperature is preferably from -78 to 50°C.

A reaction time is preferably from 10 minutes to 12 hours after addition of LDA and from 10 minutes to 12 hours after addition of N,N-dimethylformamide.

Next, at least one of Compound (C) and Compound (E) undergoes a cross-coupling reaction with Compound (F) in a reaction solvent to produce Compound (G).

The method for producing Compound (G) is not particularly limited, and Compound (G) can be produced by a method similar to a method described in a literature such as Adv. Energy Mater., 2018, Vol. 8, p. 1801212; J. Mater. Chem. C, 2020, Vol. 8, p. 15175; or ACS Energy Lett., 2019, Vol. 4, p. 1401. An example of specific producing conditions is as follows.

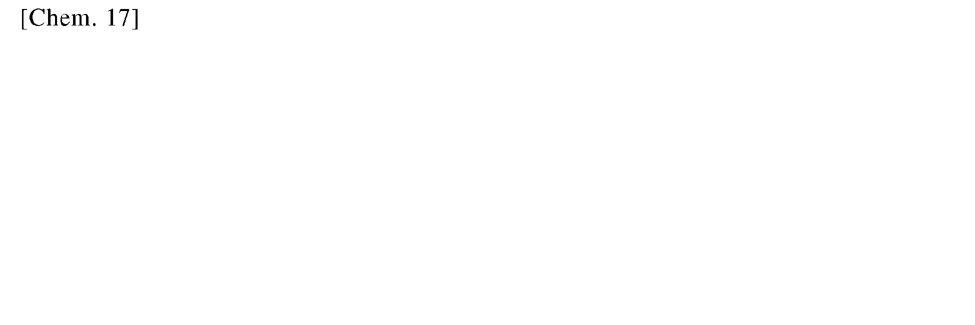

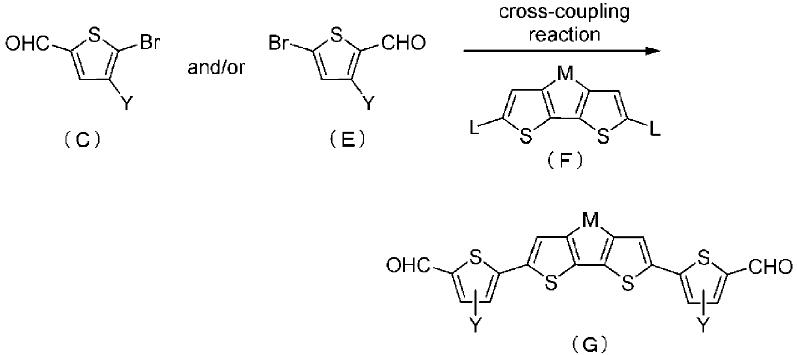

Here, Compound (F) may be synthesized by a known method (for example, Polym. Chem., 2013, Vol. 4, p. 5351-5360; J. Phys. Chem. C, 2011, Vol. 15, p. 2398-2405; and Chem. Commun., 2012, Vol. 48, p. 11130-11132), or a commercially available product may be used. In addition, Compound (F) ("L" in Compound (F) is an alkyltin group, boric acid, a borate ester group, zinc halide, magnesium halide, a silyl group, or the like) corresponding to the cross-coupling reaction may be used.

The type of the cross-coupling reaction, which is a reaction between Compound (F) and at least one of Compound (C) and Compound (E), is not particularly limited, and the reaction can be carried out by Stille coupling, Suzuki coupling, Negishi coupling, Kumada coupling, Hiyama coupling, or the like. The cross-coupling reaction may be carried out in the presence of a catalyst such as a palladium catalyst, a nickel catalyst, or a copper catalyst.

In the case of reacting Compound (C) or Compound (E) with Compound (F) by the Stille coupling reaction, the charging ratio of the raw material is preferably from 1.9 to 3.0 equivalents for Compound (C) or Compound (E) with respect to Compound (F). When Compound (C) and Compound (E) are reacted with Compound (F) by the Stille coupling reaction, the charging ratio of the raw material is preferably from 0.9 to 1.1 equivalents for each of Compound (C) and Compound (E) with respect to Compound (F). In addition, a palladium catalyst is preferably used in the Stille coupling reaction, and a content of palladium in the catalyst is preferably from 0.1 to 50 mol%.

The reaction solvent is not particularly limited as long as it is a solvent that does not react with the raw material compound, and examples thereof include saturated aliphatic hydrocarbon solvents such as hexane; ether-based solvents such as diethyl ether, cyclopentyl methyl ether, tetrahydrofuran (THF), and 1,4-dioxane; aromatic hydrocarbon solvents such as toluene and xylene; N,N-dimethylformamide (DMF), dimethyl sulfoxide, and N-methyl-2-pyrrolidone.

A reaction temperature is preferably from 20°C to the reflux temperature of the reaction solvent.

A reaction time is preferably from 1 to 24 hours.

Then, Compound (G) is reacted with Compound (I) in the presence of an acid catalyst in a reaction solvent to produce Compound (A).

The method for producing Compound (A) is not particularly limited, and Compound (A) can be produced by a method similar to a method described in JP 2022-511781 A or JP 2023-500815 A, for example. An example of specific producing conditions is as follows.

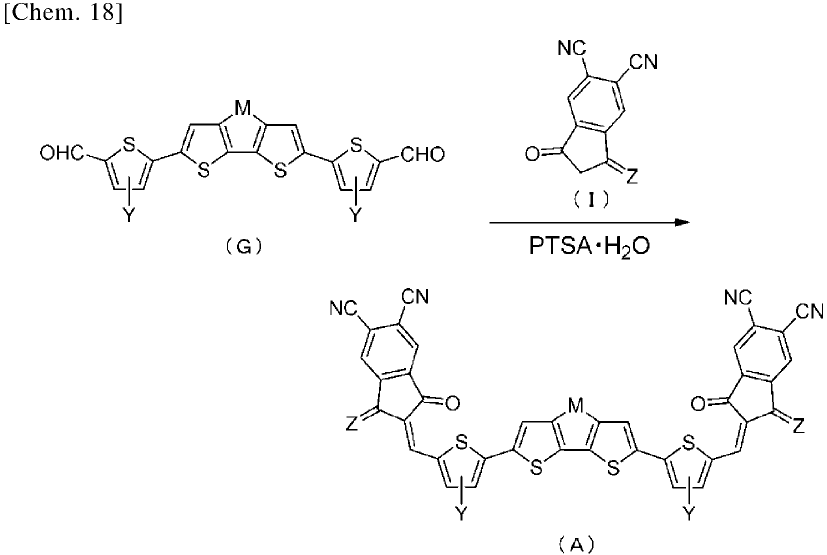

Here, Compound (I) can be synthesized by a known method (for example, JP 2022-511781 A or JP 2023-500815 A).

Examples of the acid catalyst include p-toluenesulfonic acid hydrate (PTSA·H₂O).

The charging ratio of the raw material is preferably from 1.9 to 10 equivalents for Compound (I) with respect to Compound (G). The charging ratio of the raw material is preferably from 2.1 to 11 equivalents for p-toluenesulfonic acid hydrate.

The reaction solvent is not particularly limited as long as it is a solvent that does not react with the raw material compound, and for example, an aromatic hydrocarbon solvent such as toluene or xylene may be mixed with an alcohol-based solvent such as methanol or ethanol and the resulting mixture thereof may be used.

A reaction temperature is preferably from room temperature to reflux temperature.

A reaction time is preferably from 1 to 6 hours.

In this manner, Compound (A) can be produced. Here, Y in Compound (A) is any one of Y¹ to Y⁴ in Compound (1), and Z in Compound (A) is Z¹ or Z² in Compound (1).

The compound of the present invention is suitable as an n-type semiconductor material (light absorbing material and electron transporting material) used in a photoelectric conversion element because the compound can lengthen the absorption wavelength while suppressing the dark current of the photoelectric conversion element.

The use of the compound of the present invention is not limited to the above-described uses. For example, the compound of the present invention is also excellent in luminescence, and therefore, can be used in bioimaging, organic EL, near-infrared luminescent dyes for wavelength conversion films and compositions, and the like.

### [Composition]

The composition of the present invention contains Compound (1) described above.

One type of Compound (1) may be used alone, or two or more types thereof may be used in combination and any ratio.

A content of Compound (1) in the composition of the present invention is not particularly limited. However, in a case where the composition of the present invention is used for forming a photoelectric conversion layer (active layer) of a photoelectric conversion element, the content of Compound (1) is preferably high in terms of the amount of light absorption, and on the other hand, the content is preferably low in terms of carrier balance. Therefore, the content of Compound (1) is preferably 10 mass% or more, more preferably 25 mass% or more, and still more preferably 40 mass% or more, with respect to a total amount (total mass) of all components other than the solvent in the composition of the present invention. In addition, the content of Compound (1) is preferably 100 mass% or less, more preferably 90 mass% or less, still more preferably 75 mass% or less, and particularly preferably 60 mass% or less, with respect to the total amount (total mass) of all components other than the solvent in the composition of the present invention. The above upper and lower limits can be combined in any manner. For example, the content may be from 10 to 100 mass%, from 10 to 90 mass%, from 25 to 75 mass%, or from 40 to 60 mass%.

The composition of the present invention may further contain a solvent. The composition containing Compound (1) and a solvent is suitable as an ink (active layer forming composition) for forming a photoelectric conversion layer (active layer) of a photoelectric conversion element.

The solvent is preferably a liquid that does not react with Compound (1) and dissolves Compound (1), and examples thereof include aromatic hydrocarbon solvents such as toluene and xylene; and halogen-based solvents such as dichloromethane and chloroform.

In a case where the composition of the present invention contains a solvent, one type of the solvent may be used alone, or two or more types thereof may be used in combination and any ratio.

In a case where the composition of the present invention contains a solvent, the content of Compound (1) in the composition of the present invention is preferably 0.1 mass% or more, more preferably 0.5 mass% or more, and still more preferably 1 mass% or more, with respect to the total mass of the composition of the present invention. In addition, the content of Compound (1) is preferably 5 mass% or less, more preferably 3.5 mass% or less, and still more preferably 2 mass% or less, with respect to the total mass of the composition of the present invention. The above upper and lower limits can be combined in any manner. For example, the content may be from 0.1 to 5 mass%, from 0.5 to 3.5 mass%, or from 1 to 2 mass%.

When the composition of the present invention is used as an active layer forming composition, the composition preferably further contains a p-type semiconductor material in addition to Compound (1).

The p-type semiconductor material is not particularly limited as long as it is used in a photoelectric conversion layer of an organic photoelectric conversion element, and examples thereof include polymers described in literatures (ACS Energy Lett., 2019, Vol. 4, p. 1401, and Adv. Optical Mater., 2022, Vol. 10, p. 2200747).

In a case where the composition of the present invention contains a p-type semiconductor material, one type of the p-type semiconductor material may be used alone, or two or more types thereof may be used in combination and any ratio.

The mass ratio of Compound (1) to the p-type semiconductor material (compound (1)/p-type semiconductor material) is preferably 0.1 or more, more preferably 0.5 or more, and still more preferably 0.75 or more. The mass ratio of Compound (1) to the p-type semiconductor material is preferably 3 or less, more preferably 2 or less, and still more preferably 1.5 or less. The above upper and lower limits can be combined in any manner. For example, the mass ratio may be from 0.1 to 3, from 0.5 to 2, or from 0.75 to 1.5.

The composition of the present invention may further contain a component (optional component) other than Compound (1), the p-type semiconductor material and the solvent, as necessary, within a range where the effects of the present invention are not impaired.

Examples of the optional component include 1,8-diiodooctane and 1-chloronaphthalene.

In a case where the composition of the present invention contains an optional component, one type of the optional component may be used alone, or two or more types thereof may be used in combination and any ratio.

In a case where the composition of the present invention contains an optional component, a content of the optional component is preferably high from the viewpoint that the effect of incorporating the optional component is easily exhibited. On the other hand, the content of Compound (1) is preferably high from the viewpoint that the composition of the present invention is likely to maintain physical properties suitable for a photoelectric conversion element. Therefore, in a case where the composition of the present invention contains an optional component, the content of the optional component is preferably 0.1 mass% or more, and more preferably 0.3 mass% or more, with respect to the total amount (total mass) of all components other than the solvent in the composition of the present invention. In addition, the content of the optional component is preferably 2 mass% or less, and more preferably 1 mass% or less with respect to the total amount (total mass) of all components other than the solvent in the composition of the present invention. The above upper and lower limits can be combined in any manner. For example, the content may be from 0.1 to 2 mass%, or from 0.3 to 1 mass%.

The composition of the present invention can be produced, for example, by dissolving Compound (1) and, as necessary, one or more of the p-type semiconductor material and the optional component in a solvent. The composition of the present invention containing no solvent can be produced by removing the solvent from the produced composition.

The composition of the present invention is suitable as an ink (active layer forming composition) for forming a photoelectric conversion layer (active layer) of a photoelectric conversion element.

### [Film]

The film of the present invention is a film containing Compound (1) described above, and is also referred to as organic thin film.

The film of the present invention can be produced, for example, by removing the solvent from the composition of the present invention containing the solvent described above. Specifically, the composition of the present invention containing the solvent is applied onto a base material and then dried, thereby producing the film.

A content of Compound (1) in the film is the same as the content of Compound (1) with respect to the total amount (total mass) of all components other than the solvent in the composition of the present invention described above. That is, the content of Compound (1) is preferably 10 mass% or more, more preferably 25 mass% or more, and still more preferably 40 mass% or more, with respect to the total mass of the film. The content of Compound (1) is preferably 100 mass% or less, more preferably 90 mass% or less, still more preferably 75 mass% or less, and particularly preferably 60 mass% or less, with respect to the total mass of the film. The above upper and lower limits can be combined in any manner. For example, the content may be from 10 to 100 mass%, from 10 to 90 mass%, from 25 to 75 mass%, or from 40 to 60 mass%.

A film thickness is preferably large in terms of the amount of light absorption. On the other hand, in terms of external quantum efficiency (EQE) in a case where the film of the present invention is used as a photoelectric conversion layer (active layer) of a photoelectric conversion element, the film thickness is preferably small. Therefore, the film thickness is preferably 10 nm or more, and more preferably 100 nm or more. The film thickness is preferably 2000 nm or less, and more preferably 1000 nm or less. The above upper and lower limits can be combined in any manner. For example, the film thickness may be from 10 to 2000 nm, or from 100 to 1000 nm. The film thickness can be adjusted by an application amount of the composition to be applied to the base material.

The method of applying the composition is not particularly limited, and examples thereof include brush coating, bar coating, spray coating, dip coating, spin coating, and curtain coating.

A drying temperature after the application is preferably from 20 to 250°C.

A drying time is preferably from 10 minutes to 5 hours.

The film of the present invention is suitable as a photoelectric conversion layer (active layer) of a photoelectric conversion element.

### [Photoelectric Conversion Element]

The photoelectric conversion element of the present invention is an element including the film of the present invention described above, and is also referred to as organic photoelectric conversion element. Specifically, the photoelectric conversion element of the present invention includes the film of the present invention as a photoelectric conversion layer (active layer).

A structure of the photoelectric conversion element may be a structure of a known organic photoelectric conversion element. For example, JP 2007-324587 A may be referred to. The specific structure is not particularly limited, and examples thereof include an element having a laminated structure in which a photoelectric conversion layer (active layer) is sandwiched between a pair of electrodes.

Hereinafter, an example of the photoelectric conversion element of the present invention will be described with reference to FIG. 1.

In each drawing used in the following description, to make features thereof easy to understand, portions corresponding to the features are sometimes shown enlarged for the sake of convenience and the dimensional ratios and the like of components may differ from the actual ones. Materials, dimensions, and the like exemplified in the following description are examples, and the present invention is not limited thereto, and can be appropriately modified and implemented without changing the gist of the invention.

A photoelectric conversion element 10 shown in FIG. 1 has a structure in which a transparent electrode 12, a hole transport layer 13, a photoelectric conversion layer 14, an electron transport layer 15, and a metal electrode 16 are laminated in this order on a transparent substrate 11.

The positions of the hole transport layer 13 and the electron transport layer 15 may be exchanged. That is, the photoelectric conversion element may have a structure in which a transparent electrode, an electron transport layer, a photoelectric conversion layer (active layer), a hole transport layer, and a metal electrode are laminated in this order on a transparent substrate.

The transparent substrate 11 is, for example, a base material having a mean transmission rate of 80% or more in a visible light region of 450 nm or more.

Examples of a material forming the transparent substrate 11 include glass; and plastics such as polyethylene terephthalate, polyethylene naphthalate, polycarbonate, and polyethylene sulfide.

The transparent electrode 12 is, for example, an electrode having a mean transmission rate of 80% or more in the visible light region of 450 nm or more.

A material forming the transparent electrode 12 is not particularly limited as long as the transparent electrode 12 can be formed, and examples thereof include indium oxide doped with tin (ITO), indium oxide doped with zinc (IZO), indium oxide doped with tungsten (IWO), an oxide of zinc and aluminum (AZO), indium oxide (In₂O₃), zinc oxide (ZnO), and titanium oxide (TiO₂).

The metal electrode 16 is an electrode that forms a pair with the transparent electrode 12.

A material constituting the metal electrode 16 is not particularly limited, and examples thereof include metals such as gold, platinum, silver, aluminum, nickel, titanium, magnesium, calcium, barium, sodium, chromium, copper, and cobalt, and alloys thereof.

The metal electrode 16 is preferably a transparent electrode or a reflective electrode. That is, the photoelectric conversion element preferably has a laminated structure in which a photoelectric conversion layer (active layer) is sandwiched between a pair of electrodes (transparent or metal), and more preferably has a laminated structure in which an electron transport layer, a photoelectric conversion layer (active layer), and a hole transport layer are sandwiched between a pair of electrodes (transparent or metal). In the case of a pair of transparent electrodes, materials forming the electrodes may be of the same type or different types.

A thickness of the metal electrode 16 is not particularly limited, and is preferably approximately 10 nm from the viewpoint of increasing transparency. When the transparency is not required, the thickness is preferably 40 nm or more, and more preferably 100 nm or more, for example, in consideration of endurance.

The methods for forming the transparent electrode 12 and the metal electrode 16 are not particularly limited, and the transparent electrode 12 and the metal electrode 16 can be formed by, for example, a dry process such as vacuum deposition or sputtering; or a wet process using an electroconductive ink or the like.

In the case of providing the hole transport layer 13 and the electron transport layer 15, the constituent members and the production method thereof are not particularly limited, and known techniques can be used. For example, the members and production methods thereof described in WO 2013/171517, WO 2013/180230, and JP 2012-191194 A can be used.

The photoelectric conversion layer 14 is a layer that absorbs light and separates charges.

The photoelectric conversion layer 14 of the photoelectric conversion element of the present invention is a layer containing Compound (1) of the present invention described above. More specifically, it is the film of the present invention described above.

The photoelectric conversion layer 14 can be formed by, for example, applying the composition of the present invention described above onto a layer to be under the photoelectric conversion layer 14, such as the hole transport layer 13, and drying the composition.

The photoelectric conversion element 10 can be produced, for example, by forming the transparent electrode 12, the hole transport layer 13, the photoelectric conversion layer 14, the electron transport layer 15, and the metal electrode 16 in this order on the transparent substrate 11.

Since the photoelectric conversion layer 14 contains Compound (1), the photoelectric conversion element of the present invention can lengthen the absorption wavelength while suppressing the dark current, and has high sensor sensitivity on the long wavelength side.

### [CMOS Image Sensor]

The CMOS image sensor of the present invention includes the photoelectric conversion element of the present invention described above.

A structure of the CMOS image sensor may be a structure of a known CMOS image sensor. Specifically, for example, JP 2021-57422 A may be referred to, and the structure is not particularly limited. More specifically, a CMOS image sensor having a structure in which a metal wiring, the photoelectric conversion element of the present invention, a color filter, and a microlens are laminated in this order on a substrate such as a silicon substrate is exemplified.

### Examples

Hereinafter, the present invention will be described in more detail with reference to Examples, but the following Examples do not limit the scope of the present invention.

### [Synthesis of Compound (1-1)]

Compound (G-1) was synthesized by a method described in a known literature (ACS Energy Lett., 2019, Vol. 4, p. 1401).

Separately, Compound (I-1) was synthesized by a method described in JP 2023-500815 A.

In a reaction vessel, 0.100 g (0.12 mmol) of Compound (G-1) and 0.144 g (0.59 mmol) of Compound (I-1) were placed, 2.9 mL of toluene and 5.8 mL of ethanol were added thereto for dissolution, and 0.168 g (0.89 mmol) of p-toluenesulfonic acid monohydrate was then added thereto, followed by stirring at room temperature for 3.5 hours. After allowing the solution to cool, an organic layer was extracted with ethyl acetate and water, an extract was dried over sodium sulfate, and a solvent of the solution from which solid components were removed by filtration was distilled off, resulting in the formation of a solid. The solid was purified by silica gel chromatography, and the purified solid was dispersed in methanol and filtered to afford 0.068 g of a compound as a black solid (in 44% yield).

The resulting compound was confirmed to be Compound (1-1) by¹H-NMR analysis. The ¹H-NMR measurement data is shown below.

¹H-NMR (400 MHz, solvent: CDCl₃, ppm): δ8.80 (s, 2H), 8.78 (s, 2H), 7.96-7.97 (s, 2H), 7.66 (br.s., 2H), 7.46-7.51 (m, 2H), 2.84 (d, 4H), 1.96-2.06 (m, 4H), 1.79 (br.s., 2H), 1.25-1.44 (m, 16H), 0.90-1.04 (m, 28H), 0.72-0.75 (m, 8H), 0.67 (t, 6H).

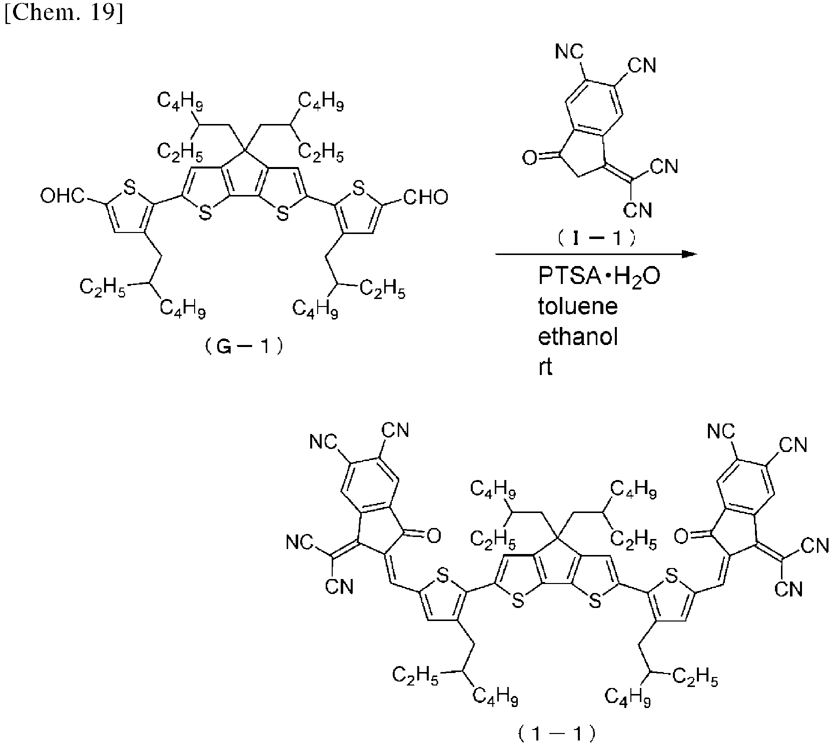

### [Synthesis of Compound (1-2)]

Compound (G-2) was synthesized by a method described in a known literature (Adv. Energy Mater., 2018, Vol. 8, p. 1801212).

Separately, Compound (1-1) was synthesized by a method described in JP 2023-500815 A.

In a reaction vessel, 0.244 g (0.29 mmol) of Compound (G-2) and 0.350 g (1.43 mmol) of Compound (I-1) were placed, 7.0 mL of toluene and 14 mL of ethanol were added thereto for dissolution, and 0.409 g (2.15 mmol) of p-toluenesulfonic acid monohydrate was then added thereto, followed by stirring at 65°C for 1 hour. After allowing the solution to cool, an organic layer was extracted with ethyl acetate and water, an extract was dried over sodium sulfate, and a solvent of the solution from which solid components were removed by filtration was distilled off, resulting in the formation of a solid. The resulting solid was dispersed in ethanol and filtered to afford 0.288 g of a compound as a black solid (in 77% yield).

The resulting compound was confirmed to be Compound (1-2) by ¹H-NMR analysis. The ¹H-NMR measurement data is shown below.

¹H-NMR (400 MHz, solvent: CDCl₃, ppm): δ8.75 (s, 2H), 8.73 (s, 2H), 7.92 (s, 2H), 7.63-7.64 (m, 2H), 7.51 (br.s., 2H), 4.17 (d, 4H), 1.87-2.03 (m, 6H), 1.57-1.69 (m, 8H), 1.40-1.42 (m, 8H), 0.89-1.05 (m, 28H), 0.70-0.74 (m, 8H), 0.65 (t, 6H).

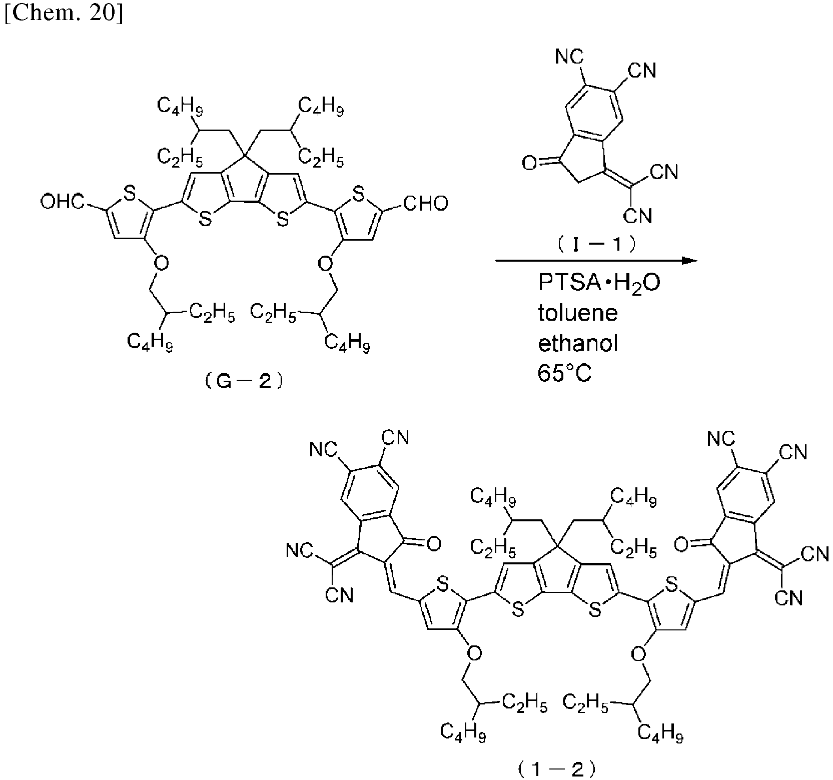

### [Synthesis of Compound (1-5)]

Compound (C-1) was synthesized by a method described in a known literature (Adv. Mater., 2016, Vol. 28, p. 8283).

Separately, Compound (E-1) was synthesized by a method described in CN 115057995 A.

Separately, Compound (F-1) was synthesized by a method described in a known literature (Macromolecules, 2007, Vol. 40, p. 1981).

In a reaction vessel, 0.219 g (0.69 mmol) of Compound (C-1), 0.229 g (0.76 mmol) of Compound (E-1), 0.500 g (0.69 mmol) of Compound (F-1), and 0.024 g (0.034 mmol) of Pd(Amphos)Cl₂ were placed, and the reaction vessel was evacuated and then purged with nitrogen. Toluene (6.3 mL) was added, followed by heating and stirring at 80°C for 2 hours. After allowing the solution to cool, the solvent was removed under reduced pressure, and the residue was then purified by silica gel column chromatography to afford 0.290 g of a compound (in 44% yield).

The resulting compound was confirmed to be Compound (G-3) by ¹H-NMR analysis. The ¹H-NMR measurement data is shown below.

¹H-NMR (400 MHz, CDCl₃, ppm): δ9.96 (s, 1H), 9.76 (s, 1H), 7.47 (s, 1H), 7.31-7.32 (m, 1H), 7.20-7.21 (m, 1H), 7.00 (s, 1H), 4.11 (d, 2H), 2.85 (d, 2H), 1.82-1.95 (m, 5H), 1.50-1.67 (m, 5H), 1.31-1.40 (m, 12H), 0.83-1.02 (m, 28H), 0.61-0.76 (m, 14H).

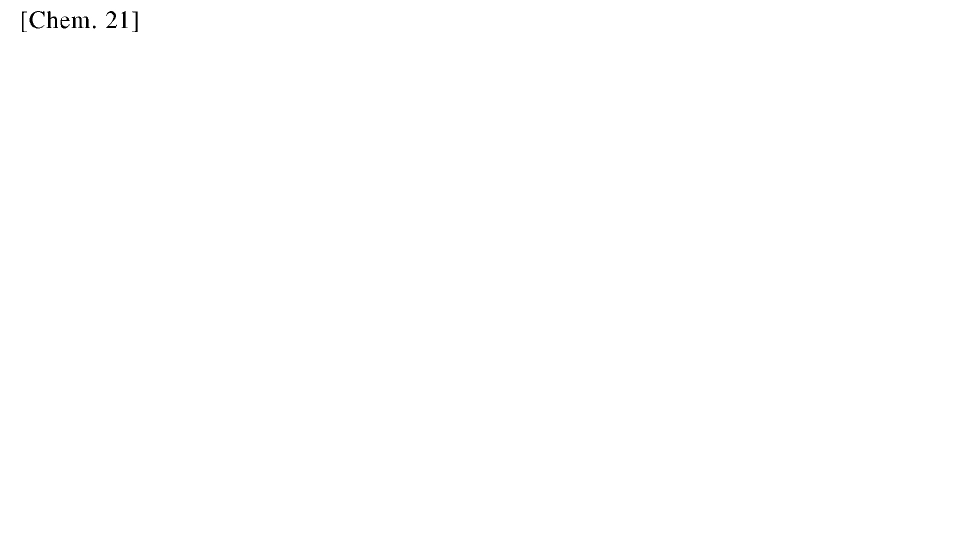

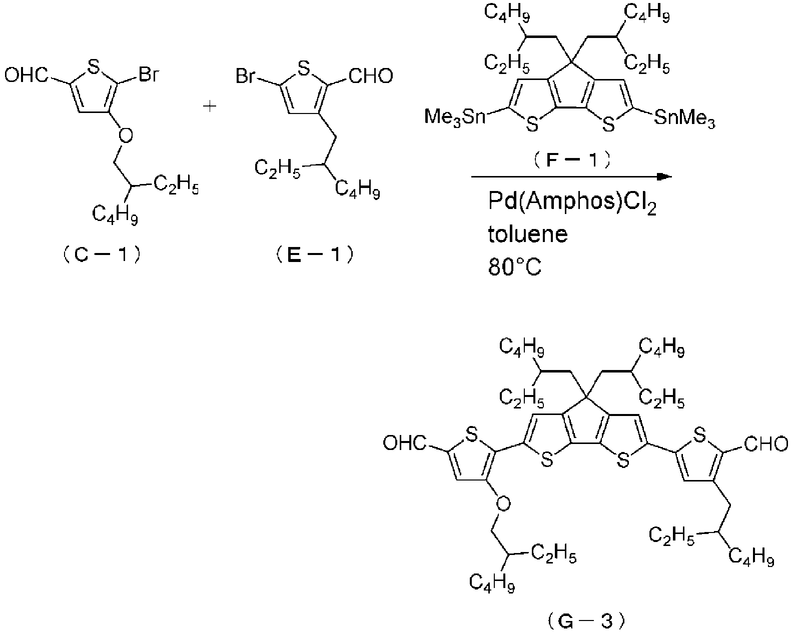

Separately, Compound (1-1) was synthesized by a method described in JP 2023-500815 A.

In a reaction vessel, 0.070 g (0.08 mmol) of Compound (G-3) and 0.10 g (0.40 mmol) of Compound (I-1) were placed, 6.0 mL of toluene and 8 mL of ethanol were added thereto for dissolution, and 0.120 g (0.60 mmol) of p-toluenesulfonic acid monohydrate was then added thereto, followed by stirring at 65°C for 3 hours. After allowing the solution to cool, an aqueous sodium hydrogen carbonate solution was poured. An organic layer was extracted by a liquid separation operation, an extract was dried over sodium sulfate, and a solvent of the solution from which solid components were removed by filtration was distilled off, resulting in the formation of a solid. The resulting solid was dispersed in methanol and filtered to afford 0.10 g of a compound as a black solid (in 92% yield).

The resulting compound was confirmed to be Compound (1-5) by ¹H-NMR analysis. The ¹H-NMR measurement data is shown below.

¹H-NMR (400 MHz, solvent: CDCl₃, ppm): δ8.98 (s, 1H), 8.77 (s, 1H), 8.75 (s, 1H), 8.73 (s, 1H), 7.94 (s, 1H), 7.93 (s, 1H), 7.63-7.64 (m, 1H), 7.52-7.53 (m, 2H), 7.24 (s, 1H), 4.17 (d, 2H), 2.92 (d, 2H), 1.87-2.04 (m, 5H), 1.53-1.74 (m, 5H), 1.28-1.41 (m, 12H), 0.87-1.05 (m, 28H), 0.64-0.73 (m, 14H).

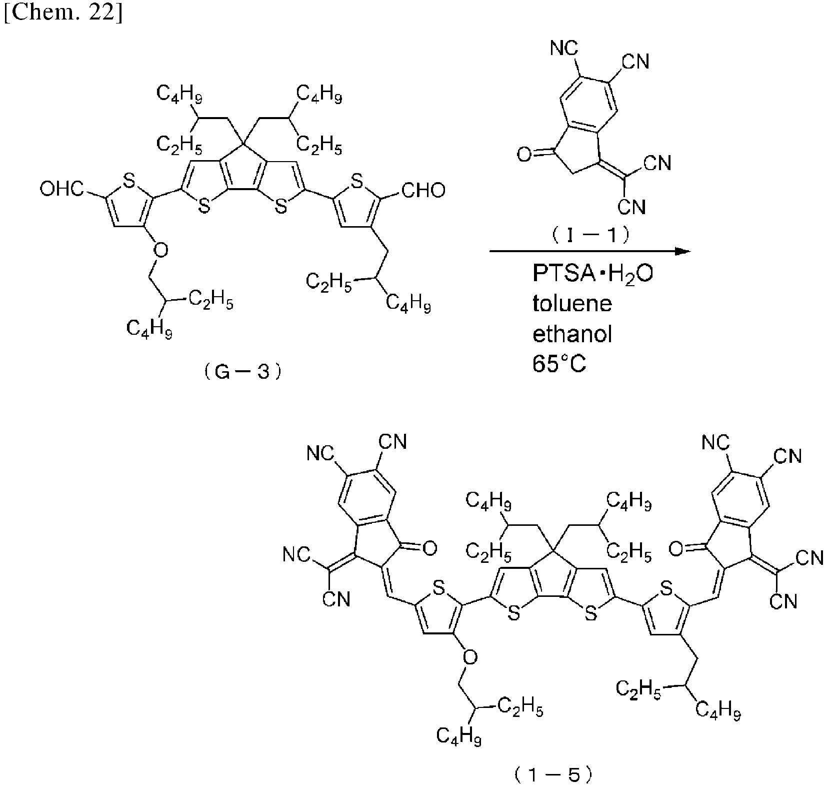

### [Synthesis of Compound (1-28)]

By a method similar to a method described in a known literature (New J. Chem., 2020, Vol. 44, p. 8032), 3-[(1-ethylhexyl)oxy]thiophene (¹H-NMR (400 MHz, solvent: CDCl₃, ppm): δ7.14 (dd, 1H), 6.74 (dd, 1H), 6.21 (dd, 1H), 4.00 (quin, 1H), 1.71-1.58 (m, 4H), 1.50-1.28 (m, 6H), 0.95 (t, 3H), 0.88 (t, 3H)) was synthesized.

Using the resulting 3-[(1-ethylhexyl)oxy]thiophene, 4-[(1-ethylhexyl)oxy]-2-thiophenecarbaldehyde (¹H-NMR (400 MHz, solvent: CDCl₃, ppm): δ9.81 (s, 1H), 7.40 (dd, 1H), 6.73 (m, 1H), 4.03 (quin, 1H), 1.72-1.57 (m, 4H), 1.45-1.29 (m, 6H), 0.96 (t, 3H), 0.87 (t, 3H)) was synthesized by a method similar to a method described in a known literature (J. Mater. Chem. A, 2020, Vol. 8, p. 5163).

Then, 4.99 g (20.7 mmol) of 4-[(1-ethylhexyl)oxy]-2-thiophenecarbaldehyde was dissolved in acetonitrile (40 mL). To the solution, 3.73 g (20.9 mmol) of N-bromosuccinimide was slowly added. After stirring at room temperature for 1 hour, water and hexane were added, and an organic layer was washed with water three times. An extract was dried over anhydrous sodium sulfate and purified by silica gel chromatography to afford a compound (in 98% yield).

The resulting compound was confirmed to be Compound (C-2) by ¹H-NMR analysis. The ¹H-NMR measurement data is shown below.

¹H-NMR (400 MHz, solvent: CDCl₃, ppm): δ9.70 (s, 1H), 7.33 (s, 1H), 4.10 (quin, 1H), 1.73-1.59 (m, 4H), 1.45-1.29 (m, 6H), 0.96 (t, 3H), 0.88 (t, 3H).

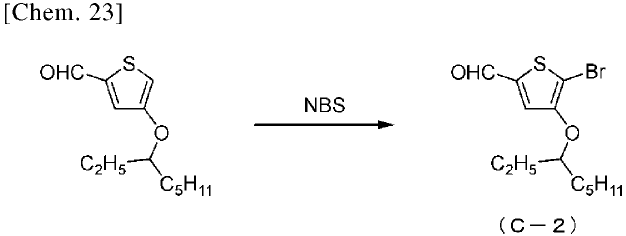

Separately, Compound (F-1) was synthesized by a method described in a known literature (Macromolecules, 2007, Vol. 40, p. 1981).

With reference to a method described in JP 2022-030124 A, 1.46 g (2.0 mmol) of Compound (F-1) was reacted with 1.35 g (4.2 mmol) of Compound (C-2). Thereafter, the solvent was removed under reduced pressure, and the residue was then purified by silica gel column chromatography to afford a compound (in 85% yield).

The resulting compound was confirmed to be Compound (G-4) by ¹H-NMR analysis. The ¹H-NMR measurement data is shown below.

¹H-NMR (400 MHz, solvent: CDCl₃, ppm): δ9.74 (s, 2H), 7.44 (s, 2H), 7.34 (s, 2H), 4.31 (quin, 2H), 1.97-0.61 (m, 66H).

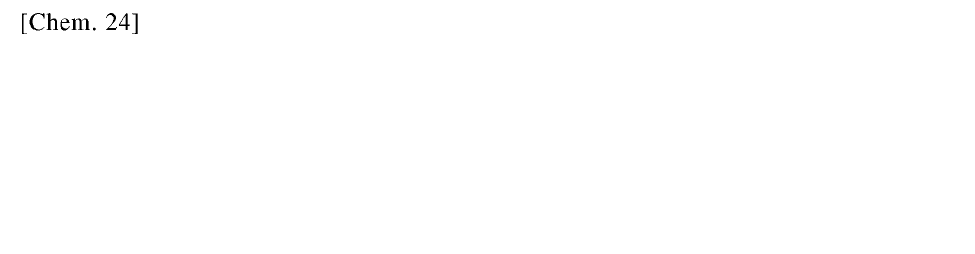

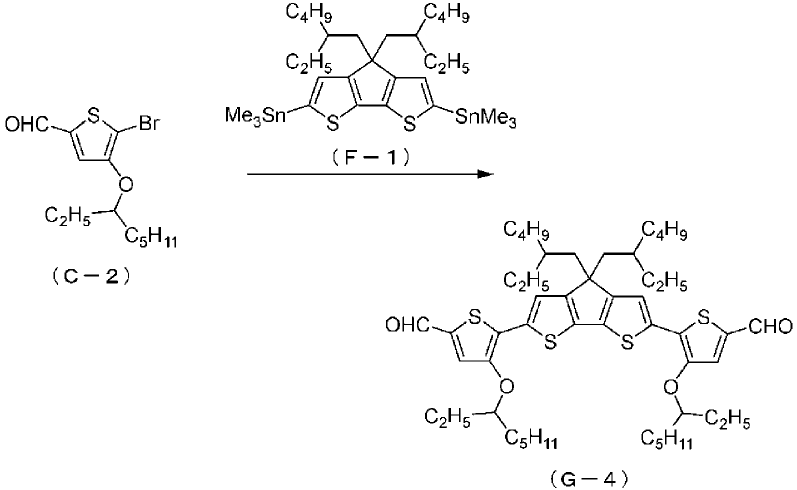

Separately, Compound (1-1) was synthesized by a method described in JP 2023-500815 A.

In a reaction vessel, 0.294 g (0.33 mmol) of Compound (G-4) and 0.408 g (1.67 mmol) of Compound (I-1) were placed, 8.2 mL of toluene and 16.3 mL of ethanol were added thereto for dissolution, and 0.477 g (2.51 mmol) of p-toluenesulfonic acid monohydrate was then added thereto, followed by stirring at 65°C for 2 hours. After allowing the solution to cool, an organic layer was extracted with ethyl acetate and water, an extract was dried over sodium sulfate, and a solvent of the solution from which solid components were removed by filtration was distilled off, resulting in the formation of a solid. The solid was dispersed in methanol and filtered to afford 0.30 g of a compound (in 67% yield).

The resulting compound was confirmed to be Compound (1-28) by ¹H-NMR analysis. The ¹H-NMR measurement data is shown below.

¹H-NMR (400 MHz, solvent: CDCl₃, ppm): δ8.74 (s, 2H), 8.69 (s, 2H), 7.92 (s, 2H), 7.61-7.62 (m, 2H), 7.50 (br.s., 2H), 4.41-4.44 (quin, 2H), 1.76-2.03 (m, 12H), 1.45-1.51 (br.s., 4H), 1.33-1.37 (m, 8H), 0.89-1.10 (m, 28H), 0.63-0.74 (m, 14H).

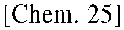

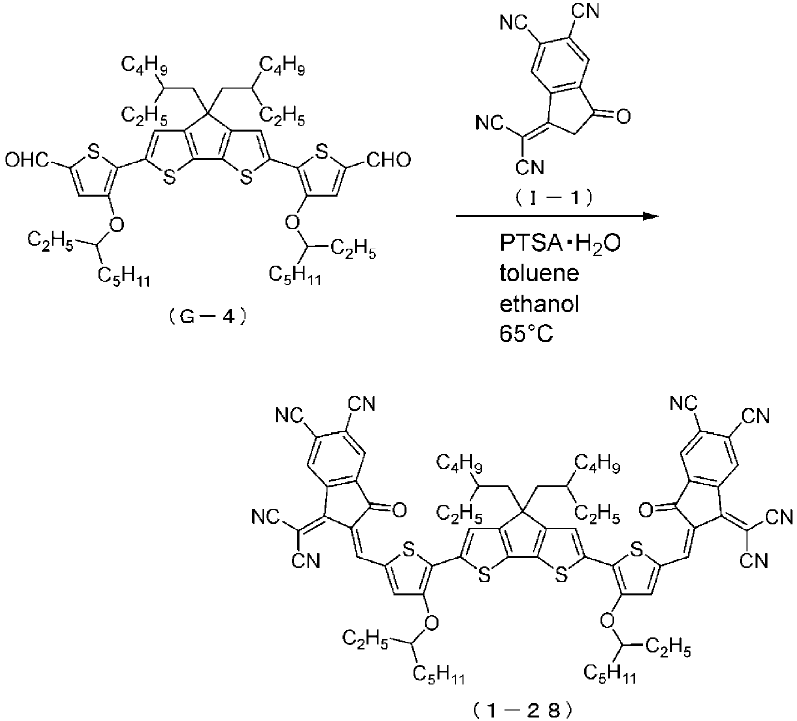

### [Synthesis of Compound (N-2)]

Compound (G-3) was synthesized in the same manner as in the synthesis of compound (1-5).

In a reaction vessel, 0.200 g (0.23 mmol) of Compound (G-3), 0.150 g (0.56 mmol) of Compound (I-1) commercially available, and 5.7 mL of chloroform were placed, stirred and dissolved, and 0.2 mL of pyridine was then added thereto, followed by stirring at 60°C for 4 hours. After allowing the solution to cool, chloroform was distilled off, and methanol was added instead to disperse the solid, followed by filtration. The resulting solid was purified by silica gel chromatography to afford 0.140 g of a compound (in 45% yield).

The resulting compound was confirmed to be Compound (N-2) by ¹H-NMR analysis. The ¹H-NMR measurement data is shown below.

¹H-NMR (400 MHz, CDCl₃, ppm): δ8.98 (s, 1H), 8.78 (s, 1H), 8.75 (s, 1H), 8.73 (s, 1H), 7.94 (s, 1H), 7.93 (s, 1H), 7.62-7.64 (m, 1H), 7.51-7.53 (s, 2H), 7.24 (s, 1H), 4.17 (d, 2H), 2.92 (d, 2H), 1.87-2.03 (m, 5H), 1.50-1.73 (m, 5H), 1.30-1.41 (m, 12H), 0.87-1.05 (m, 28H), 0.64-0.73 (m, 14H).

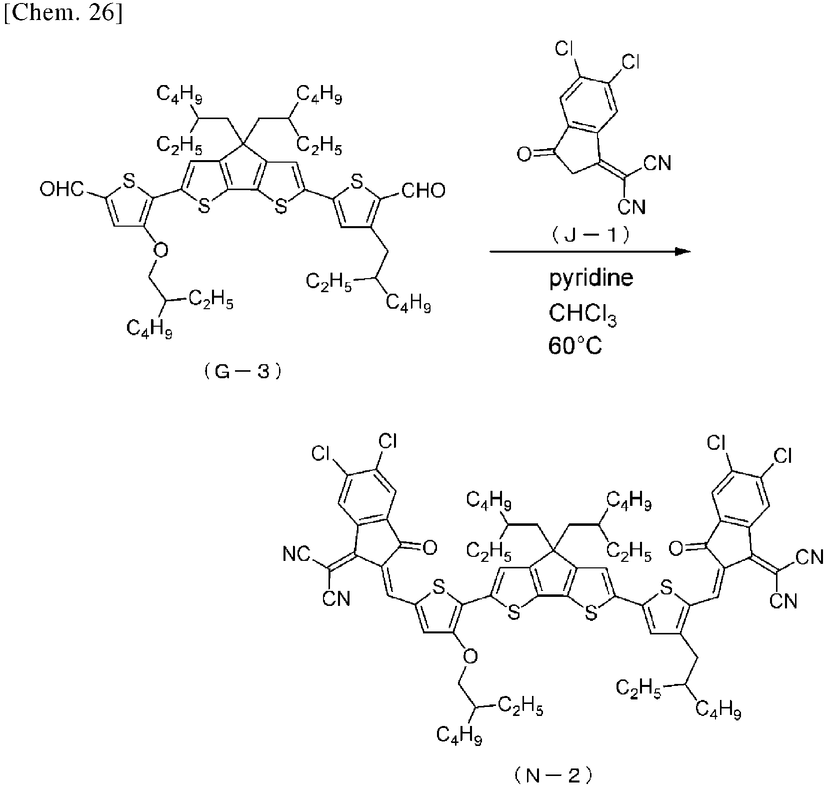

### [Example 1]

### <Production of Photoelectric Conversion Element>

### (Formation of Hole Transport Layer)

A surface of an ITO substrate having a transparent electroconductive film of indium tin oxide (ITO) as a transparent electrode pattern-formed on a glass substrate as a transparent substrate was subjected to an ozone treatment for 10 minutes using an ultraviolet ozone cleaner (product name: "NL-UV253" available from Japan Laser Electronics Co., Ltd.).

Separately, 60 mg of a polytriarylamine compound (hole transporting polymer) represented by Formula (H-1) was dissolved in 1 mL of anisole to prepare a hole transport layer forming composition.

The hole transport layer forming composition was spin-coated onto the transparent electrode of the ITO substrate after the ozone treatment at a rotation speed of 1000 rpm for 60 seconds, and heated and dried at 240°C for 30 minutes to form a hole transport layer having a thickness of 300 nm.

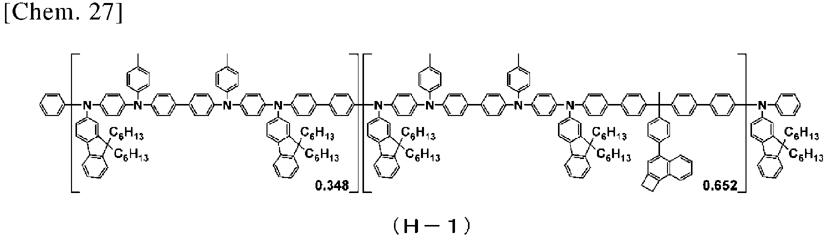

### (Formation of Photoelectric Conversion Layer)

As the p-type semiconductor material, a compound (weight average molecular weight: 80000) represented by Formula (P-1) was used.

Compound (1-2) was used as the n-type semiconductor material.

0.11 g of the p-type semiconductor material and 0.13 g of the n-type semiconductor material were dissolved in 9.68 mL of o-xylene to prepare an active layer forming composition as an organic semiconductor ink. In the active layer forming composition, the mass ratio of the n-type semiconductor material to the p-type semiconductor material (n-type semiconductor material/p-type semiconductor material) was 1.2. Also, a solid component concentration of the active layer forming composition was 25 mg/mL.

The resulting active layer forming composition was used to be spin-coated onto the hole transport layer at 1000 rpm per minute, and then subjected to a heating treatment (thermal annealing treatment) at 120°C for 10 minutes to form a photoelectric conversion layer (active layer) made of an organic thin film having a thickness of 150 nm.

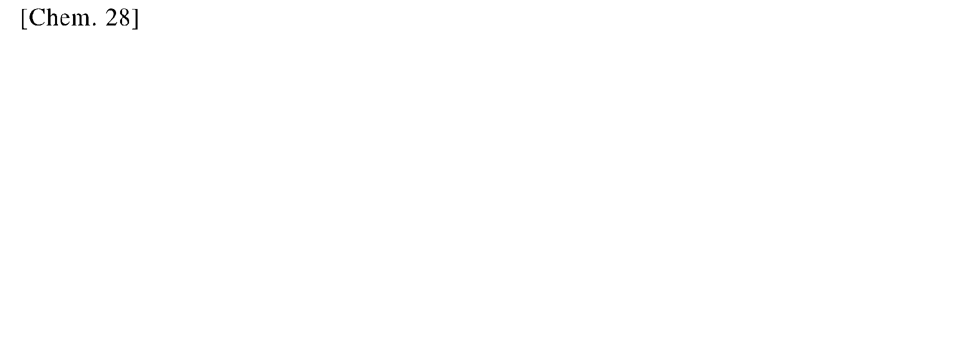

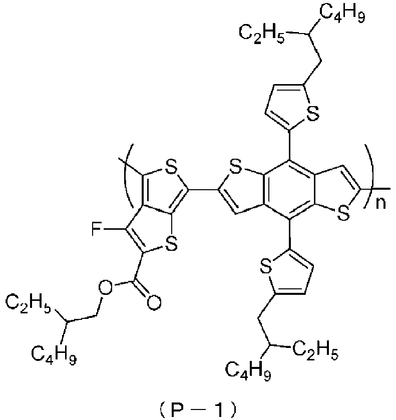

### (Formation of Electron Transport Layer and Metal Electrode)

On the photoelectric conversion layer, C60 fullerene (available from Frontier Carbon Corporation) as an electron transporting material was formed into a film in vacuum to form an electron transport layer having a thickness of 40 nm.

Next, on the electron transport layer, aluminum as a material for a metal electrode was formed into a film in vacuum to form a metal electrode having a thickness of 100 nm, thereby a photoelectric conversion element was produced.

The resulting photoelectric conversion element was evaluated as follows.

### <Evaluation>

### (Evaluation of External Quantum Efficiency (EQE))

The photoelectric conversion element was irradiated with a xenon lamp under application of -5 V, and the external quantum efficiency was measured using an action spectrum measurement apparatus (product name: "PEC-S20" available from Peccell Technologies, Inc.). The results of the external quantum efficiency at a wavelength of 1100 nm are shown in Table 1.

The values shown in Table 1 are relative values (relative EQE values) when the external quantum efficiency at the wavelength of 1100 nm of the photoelectric conversion element produced in Comparative Example 1 described later is set to 1.0.

### (Measurement of Dark Current)

The dark current when applying -5 V was measured using the photoelectric conversion element. A high-precision current measurement apparatus (product name: "Keithley 6482" available from Keithley Instruments, Inc.) was used for the measurement of the dark current. The results are shown in Table 1.

The values shown in Table 1 are relative values (relative dark current values) when the dark current of the photoelectric conversion element produced in Comparative Example 1 described later is set to 1.00.

### [Example 2]

A photoelectric conversion element was produced in the same manner as in Example 1 except that Compound (1-28) was used as the n-type semiconductor material, and the external quantum efficiency when applying -5 V was measured. The results of the external quantum efficiency at a wavelength of 1100 nm are shown in Table 1. The values shown in Table 1 are relative values (relative EQE values) when the external quantum efficiency at the wavelength of 1100 nm of the photoelectric conversion element produced in Comparative Example 1 described later is set to 1.0.

The dark current of the resulting photoelectric conversion element when applying -5 V was measured in the same manner as in Example 1. The results are shown in Table 1. The values shown in Table 1 are relative values (relative dark current values) when the dark current of the photoelectric conversion element produced in Comparative Example 1 described later is set to 1.00.

### [Comparative Example 1]

A photoelectric conversion element was produced in the same manner as in Example 1 except that a compound represented by Formula (N-1) was used as the n-type semiconductor material, and the external quantum efficiency when applying -5 V and the dark current when applying -5 V were measured.

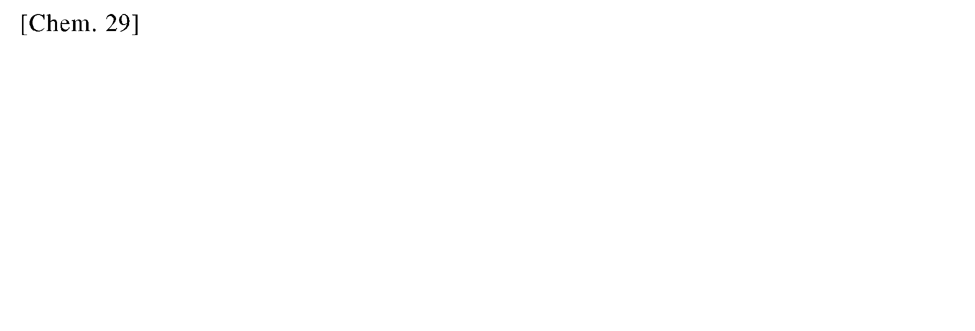

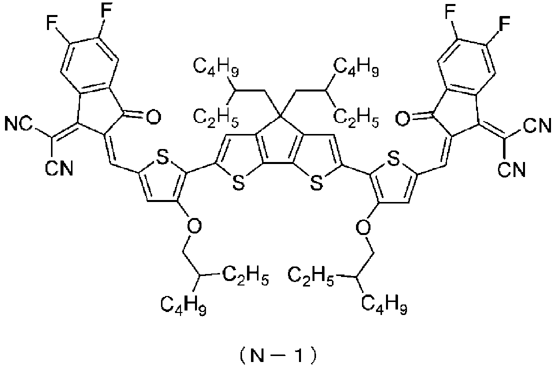

**[Table 1]**

| | n-Type semiconductor material | Relative EQE value (1100 nm light) | Relative dark current value (when applying -5 V) |
|---|---|---|---|
| Example 1 | 1-2 | 2.7 | 0.28 |
| Example 2 | 1-28 | 2.4 | 0.18 |
| Comparative Example 1 | N-1 | 1.0 | 1.00 |

The results shown in Table 1 shows that the photoelectric conversion elements produced in Examples 1 and 2 had a significantly higher EQE at 1100 nm, which was 2.7 times or 2.4 times that of the photoelectric conversion element produced in Comparative Example 1, and had high sensor sensitivity on the longer wavelength side. In addition, the photoelectric conversion elements produced in Examples 1 and 2 had a dark current as low as 0.28 times or 0.18 times that of the photoelectric conversion element produced in Comparative Example 1, and had significantly low noise.

These results showed that Compound (1) of the present invention can achieve both high sensor sensitivity in the long wavelength region of the absorption wavelength and a low dark current reduction.

### [Example 3]

A photoelectric conversion element was produced in the same manner as in Example 1 except that Compound (1-5) was used as the n-type semiconductor material, and the external quantum efficiency when applying -5 V was measured. The results of the external quantum efficiency at a wavelength of 940 nm are shown in Table 2. The values shown in Table 2 are relative values (relative EQE values) when the external quantum efficiency at the wavelength of 940 nm of the photoelectric conversion element produced in Comparative Example 2 described later is set to 1.0.

The dark current of the resulting photoelectric conversion element when applying -5 V was measured in the same manner as in Example 1. The results are shown in Table 2. The values shown in Table 2 are relative values (relative dark current values) when the dark current of the photoelectric conversion element produced in Comparative Example 2 described later is set to 1.00.

### [Comparative Example 2]

A photoelectric conversion element was produced in the same manner as in Example 1 except that Compound (N-2) was used as the n-type semiconductor material, and the external quantum efficiency when applying -5 V and the dark current when applying -5 V were measured.

**[Table 2]**

| | n-Type semiconductor material | Relative EQE value (940 nm light) | Relative dark current value (when applying -5 V) |
|---|---|---|---|
| Example 3 | 1-5 | 1.1 | 0.80 |
| Comparative Example 2 | N-2 | 1.0 | 1.00 |

The results shown in Table 2 shows that the photoelectric conversion element produced in Example 3 had a significantly higher EQE at 940 nm, which was 1.1 times that of the photoelectric conversion element produced in Comparative Example 2, and had high sensor sensitivity on the longer wavelength side. In addition, the photoelectric conversion element produced in Example 3 had a dark current as low as 0.80 times that of the photoelectric conversion element produced in Comparative Example 2, and had significantly low noise.

These results showed that Compound (1) of the present invention can achieve both high sensor sensitivity in the long wavelength region of the absorption wavelength and a low dark current reduction.

### Industrial Applicability

The compound of the present invention can lengthen an absorption wavelength while suppressing a dark current of a photoelectric conversion element, and is useful as a semiconductor material used in the photoelectric conversion element.

### Reference Signs List

10 Photoelectric conversion element
11 Transparent substrate
12 Transparent electrode
13 Hole transport layer
14 Photoelectric conversion layer
15 Electron transport layer
16 Metal electrode

## Claims

1. A compound represented by General Formula (1): 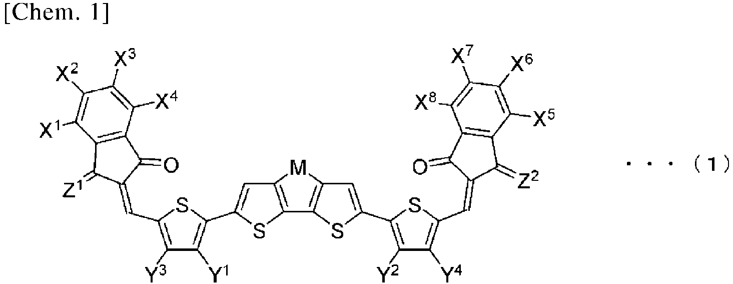 where
X¹ to X⁸ are each independently a hydrogen atom, a chlorine atom, a fluorine atom, a bromine atom, or a cyano group, and at least one of X¹ to X⁸ is a cyano group,
Y¹ to Y⁴ are each independently a hydrogen atom, an alkyl group, an alkoxy group, or an ester group, and at least one of Y¹ to Y⁴ is an alkyl group, an alkoxy group, or an ester group,
Z¹ and Z² are each independently an oxygen atom or a dicyanomethylene group, and
M is a carbon atom substituted with an alkyl group or an aryl group, a silicon atom substituted with an alkyl group or an aryl group, or a germanium atom substituted with an alkyl group or an aryl group.

2. The compound according to claim 1, wherein, in General Formula (1), Z¹ and Z² are dicyanomethylene groups.

3. The compound according to claim 1, wherein, in General Formula (1),
X¹, X⁴, X⁵ and X⁸ are hydrogen atoms, and
X², X³, X⁶ and X⁷ are cyano groups.

4. The compound according to claim 1, wherein, in General Formula (1), M is a carbon atom substituted with an alkyl group or an aryl group.

5. The compound according to claim 1, wherein, in General Formula (1),
one of Y¹ and Y³ is a hydrogen atom and the other is an alkyl group, an alkoxy group, or an ester group, and
one of Y² and Y⁴ is a hydrogen atom and the other is an alkyl group, an alkoxy group, or an ester group.

6. The compound according to claim 1, wherein, in General Formula (1),
Y³ and Y⁴ are hydrogen atoms, and
Y¹ and Y² are each independently an alkyl group, an alkoxy group, or an ester group.

7. The compound according to claim 1, wherein, in General Formula (1),
X¹, X⁴, X⁵ and X⁸ are hydrogen atoms,
X², X³, X⁶ and X⁷ are cyano groups,
Y³ and Y⁴ are hydrogen atoms,
Y¹ and Y² are alkoxy groups,
Z¹ and Z² are dicyanomethylene groups, and
M is a carbon atom substituted with an alkyl group.

8. The compound according to claim 1, wherein, in General Formula (1),
Y² and Y³ are hydrogen atoms, and
Y¹ and Y⁴ are each independently an alkyl group, an alkoxy group, or an ester group.

9. The compound according to claim 1, wherein, in General Formula (1),
X¹, X⁴, X⁵ and X⁸ are hydrogen atoms,
X², X³, X⁶ and X⁷ are cyano groups,
Y² and Y³ are hydrogen atoms,
Y¹ and Y⁴ are each independently an alkyl group or an alkoxy group,
Z¹ and Z² are dicyanomethylene groups, and
M is a carbon atom substituted with an alkyl group.

10. A composition comprising the compound described in any one of claims 1 to 9.

11. A film comprising the compound described in any one of claims 1 to 9.

12. A photoelectric conversion element comprising the film described in claim 11.

13. A CMOS image sensor comprising the photoelectric conversion element described in claim 12.
